# EUROPEAN PATENT APPLICATION

(11) **EP 2 594 264 A1**
(43) Date of publication of application: **22.05.2013**
(21) Application number: 12194855.8
(22) Date of filing: 23.02.2006
(51) Int. Cl.: A61K 31/00, A61K 31/416, A61K 31/423, A61P 27/02, A61K 31/42, A61K 31/5377

(54) **Methods for treating ocular angiogenesis, retinal edema, retinal ischemia, and diabetic retinopathy using selective rtk inhibitors**

(30) Priority: 23.02.2005 US 655676 P
(62) Divisional of application: 06736002.4
(71) Applicant: Novartis AG, 4056 Basel (CH)
(72) Inventor: Bingaman, David, P, Weatherford, TX Texas 76088 (US)
(74) Representative: Gates, Marie Christina Esther

(57) **Abstract**

The present invention provides compositions and methods for treating ocular neovascularization, angiogenesis, retinal edema, diabetic retinopathy, and/or retinal ischemia in order to prevent the loss of visual acuity associated with such conditions. More specifically, the present invention provides compositions containing receptor tyrosine kinase (RTK) inhibitors having unique binding profiles and their use in treating ocular disorders.

## Description

This application claims priority from the provisional application, U.S. Patent Application Serial No. 60/655,676 filed February 23, 2005.

### BACKGROUND OF THE INVENTION

### 1. Field of the Invention

The present invention is directed to the prevention and treatment of ocular neovascularization, angiogenesis, retinal edema, diabetic retinopathy, and sequela associated with retinal ischemia. In particular, the present invention is directed to the use of selective Receptor Tyrosine Kinase inhibitors (RTKi's) to treat such disorders.

### 2. Description of the Related Art

Exudative age-related macular degeneration (AMD) and proliferative diabetic retinopathy (PDR) are the major causes of acquired blindness in developed countries and are characterized by pathologic posterior segment neovascularization. The posterior segment neovascularization (PSNV) found in exudative AMD is characterized as pathologic choroidal NV, whereas PDR exhibits preretinal NV. Pathologic ocular angiogenesis, which includes PSNV, occurs as a cascade of events that progress from an initiating stimulus to the formation of abnormal new capillaries. The inciting cause in both exudative AMD and PDR is still unknown, however, the elaboration of various proangiogenic growth factors appears to be a common stimulus. Soluble growth factors, such as vascular endothelial growth factor (VEGF), platelet-derived growth factor (PDGF), basic fibroblast growth factor (bFGF or FGF-2), insulin-lilce growth factor 1 (IGF-1), angiopoietins, etc., have been found in tissues and fluids removed from patients with pathologic ocular angiogenesis. Following initiation of the angiogenic cascade, the capillary basement membrane and extracellular matrix are degraded and capillary endothelial cell proliferation and migration occur. Endothelial sprouts anastomose to form tubes with subsequent patent lumen formation. The new capillaries commonly have increased vascular permeability or leakiness due to immature barrier function, which can lead to tissue edema. Differentiation into a mature capillary is indicated by the presence of a continuous basement membrane and normal endothelial junctions between other endothelial cells and pericytes; however, this differentiation process is often impaired during pathologic conditions.

Although PSNV is the vision-threatening pathology responsible for the two most common causes of acquired blindness, treatment strategies are few and palliative at best. Approved treatments for the PSNV in exudative AMD include laser photocoagulation and photodynamic therapy with Visudyne^{®}; both therapies involve laser-induced occlusion of affected vasculature and are associated with localized laser-induced damage to the retina. For patients with PDR, grid or panretinal laser photocoagulation and surgical interventions, such as vitrectomy and removal of preretinal membranes, are the only options currently available. Several different compounds are being evaluated clinically for the pharmacologic treatment of PSNV, including RETAANE® (Alcon Research, Ltd.), Lucentis^{®} (Genentech), adPEDF (GenVeC), squalamine (Genaera), CA4P (OxiGENE), VEGF trap (Regeneron), anti-VEGF or VEGFR RNAi (Acuity and SIRNA, respectively), and LY333531 (Lilly). Macugen® (Eyetech/Pfizer), an anti-VEGF aptamer injected intravitreally, has recently been approved for such use.

Macular edema is the major cause of vision loss in diabetic patients, whereas preretinal neovascularization (PDR) is the major cause of legal blindness. Diabetes mellitus is characterized by persistent hyperglycemia that produces reversible and irreversible pathologic changes within the microvasculature of various organs. Diabetic retinopathy (DR), therefore, is a retinal microvascular disease that is manifested as a cascade of stages with increasing levels of severity and worsening prognoses for vision. Major risk factors reported for developing diabetic retinopathy include the duration of diabetes mellitus, quality of glycemic control, and presence of systemic hypertension. DR is broadly classified into 2 major clinical stages: nonproliferative diabetic retinopathy (NPDR) and proliferative diabetic retinopathy (PDR), where the term "proliferative" refers to the presence of preretinal neovascularization as previously stated.

Nonproliferative diabetic retinopathy (NPDR) and subsequent macular edema are associated, in part, with retinal ischemia that results from the retinal microvasculopathy induced by persistent hyperglycemia. NPDR encompasses a range of clinical subcategories which include initial "background" DR, where small multifocal changes are observed within the retina (e.g., microaneurysms, "dot-blot" hemorrhages, and nerve fiber layer infarcts), through preproliferative DR, which immediately precedes the development of PNV. The histopathologic hallmarks of NPDR are retinal microaneurysms, capillary basement membrane thickening, endothelial cell and pericyte loss, and eventual capillary occlusion leading to regional ischemia. Data accumulated from animal models and empirical human studies show that retinal ischemia is often associated with increased local levels of proinflammatory and/or proangiogenic growth factors and cytokines, such as prostaglandin E₂, vascular endothelial growth factor (VEGF), insulin-like growth factor-1 (IGF-1), Angiopoietin 2, etc. Diabetic macular edema can be seen during either NPDR or PDR, however, it often is observed in the latter stages of NPDR and is a prognostic indicator of progression towards development of the most severe stage, PDR.

Today, no pharmacologic therapy is approved for the treatment of NPDR and/or macular edema. The current standard of care is laser photocoagulation, which is used to stabilize or resolve macular edema and retard the progression toward PDR. Laser photocoagulation may reduce retinal ischemia by destroying healthy tissue and thereby decreasing metabolic demand; it also may modulate the expression and production of various cytokines and trophic factors. Similar to the exudative AMD treatments, laser photocoagulation in diabetic patients is a cytodestructive procedure and the visual field of the treated eye is irreversibly compromised. Other than diabetic macular edema, retinal edema can be observed in various other posterior segment diseases, such as posterior uveitis, branch retinal vein occlusion, surgically induced inflammation, endophthalmitis (sterile and non-sterile), scleritis, and episcleritis, etc.

An effective pharmacologic therapy for pathologic ocular angiogenesis, retinal edema, DR, and retinal ischemia, would provide substantial benefit to the patient, thereby avoiding invasive surgical or damaging laser procedures. Effective treatment of these pathologies would improve the patient's quality of life and productivity within society. Also, societal costs associated with providing assistance and health care to the visually impaired could be dramatically reduced.

### SUMMARY OF THE INVENTION

The present invention overcomes these and other drawbacks of the prior art by providing highly potent and efficacious prevention of pathologic ocular angiogenesis, retinal edema, diabetic retinopathy, and sequela associated with retinal ischemia, as well as inducing the regression of posterior segment neovascularization and/or angiogenesis. In one aspect, the methods of the invention include treating such disorders by administering to a patient in need thereof a composition comprising a therapeutically effective amount of a receptor tyrosine kinase inhibitor that blocks tyrosine autophosphorylation of VEGF receptor 1 (Flt-1), VEGF receptor 2 (KDR), VEGF receptor 3 (Flt-4), Tie-2, PDGFR, c-KIT, Flt-3, and CSF-1R. Preferably, the compound used in the methods of the invention will exhibit an IC₅₀ value of from 0.1 nM to 250 nM for each of these receptors. More preferably, the compound will exhibit an IC₅₀ value of from 0.1 nM to 100 nM for at least six of these receptors. Most preferably, the compound will exhibit an IC₅₀ value of less than 10 nM for at least four of these receptors.

As used herein, the phrases "each of these receptors", "each receptor listed in claim n", "at least six (or four) of these receptors", and "at least six (or four) receptors listed in claim n", describe the IC₅₀ value of each individual receptor in the list referred to. For example, in the paragraph above, the phrase "from 0.1 nM to 250 nM for each of these receptors," requires that the VEGF receptor 1 (Flt-1) have an IC₅₀ value between 0.1 nM and 250 nM, that VEGF receptor 2 (KDR) have an IC₅₀ value between 0.1 nM and 250 nM, that VEGF receptor 3 (Flt-4) have an IC₅₀ value between 0.1 nM and 250 nM, that Tie-2 have an IC₅₀ value between 0.1 nM and 250 nM, that PDGFR have an IC₅₀ value between 0.1 nM and 250 nM, that c-KIT have an IC₅₀ value between 0.1 nM and 250 nM, that Flt-1 have an IC₅₀ value between 0.1 nM and 250 nM, and that CSF-1R have an IC₅₀ value between 0.1 nM and 250 nM. Likewise, the phrase "from 0.1 nM to 100 nM for at least six of these receptors" requires that six of the eight receptors in the list referred to will each have and IC₅₀ of from 0.1 nM to 100 nM.

Occasionally herein, the phrase "simultaneously blocks tyrosine autophosphorylation" will be used to refer to the receptor binding activity of preferred compounds for use in the methods of the invention. Use of this phrase refers to the fact that preferred compounds will exhibit antagonist activity at multiple tyrosine kinase receptor subtypes. That is, they are not selective for one receptor, but are highly potent antagonists of two or more tyrosine kinase receptors.

It is important that the compounds for use in the methods of the invention exhibit a receptor binding profile where multiple receptors in the RTK family are blocked by a single compound. One preferred group of receptors for which tyrosine autophosphorylation is blocked is listed above. Additional preferred binding profiles include the following: a) Tie-2, PDGFR, and VEGF receptor 2 (KDR); b) VEGF receptor 2 (KDR), VEGF receptor 1 (Flt-1), PDGFR, and Tie-2; c) .VEGF receptor 2 (KDR), VEGF receptor 1 (Flt-1), and Tie-2; d) VEGF receptor 2 (KDR), VEGF receptor 1 (Flt-1), and PDGFR; e) VEGF receptor 2 (KDR) and Tie-2; f) VEGF receptor 2 (KDR) and PDGFR; and g) VEGF receptor 2 (KDR), Tie-2, and PDGFR.

In one preferred aspect, for each grouping of receptors listed in a)-f) above, the IC₅₀ value of each receptor in each group will be from 0.1 nM to 200 nM. In another preferred aspect, the IC₅₀ value of each receptor in each group will be from 0.1 nM to 100 nM. In yet another preferred embodiment, at least one receptor in each preferred group of receptors listed in a)-f) above will exhibit an IC₅₀ value of less than 10 nM. In yet another preferred embodiment, two or more receptors in each preferred group of receptors listed in a)-f) above will exhibit an IC₅₀ value of less than 10 nM.

Preferred receptor tyrosine kinase inhibitors for use in the methods of the invention include, but are not limited to, the following compounds:
N-[4-(3-amino-1,2-benzisoxazol-4-yl)phenyl]-N'-(3-methylphenyl)urea;
N-[4-(3-amino-1,2-benzisoxazol-4-yl)phenyl]-N'-[2-(trifluoromethyl)phenyl]urea;
N-[4-(3-amino-1,2-benzisoxazol-4-yl)phenyl]-N'-(2-fluoro-5-methylphenyl)urea;
N-[4-(3-amino-1,2-benzisoxazol-4-yl)phenyl]-N'-[3-(trifluoromethyl)phenyl]urea;
N-[4-(3-amino-1,2-benzisoxazol-4-yl)phenyl]-N'-[2-fluoro-5-(trifluoromethyl)phenyl] urea;
N-[4-(3-amino-7-methoxy-1,2-benzisoxazol-4-yl)phenyl]-N'-[2-fluoro-5-(trifluoromethyl)phenyl]urea;
N-[4-(3-amino-7-methoxy-1,2-benzisoxazol-4-yl)phenyl]-N'-(3-methylphenyl)urea;
N-[4-(3-amino-7-methoxy-1,2-benzisoxazol-4-yl)phenyl]-N'-[3-(trifluoromethyl)phenyl]urea;
N-[4-(3-amino-7-methoxy-1,2-benzisoxazol-4-yl)phenyl]-N'-(3-chlorophenyl)urea;
N-[4-(3-amino-7-methoxy-1,2-benzisoxazol-4-yl)phenyl]-N'-(2-fluoro-5-methylphenyl)urea;
N-{4-[3-ammo-7-(4-morpholinylmethyl)-1,2-benzisoxazol-4-yl]phenyl}-N'-[2-fluoro-5-(trifluoromethyl)phenyl]urea;
N-{4-[3-amino-7-(4-morpholinylmethyl)-1,2-benzisoxazol-4-yl]phenyl}-N'-[3-(trifluoromethyl)phenyl]urea;
N-{4-[3-amino-7-(4-morpholinylmethyl)-1,2-benzisoxazol-4-yl]phenyl}-N'-(3-chlorophenyl)urea;
N-{4-[3-ammo-7-(4-morpholmylmethyl)-1,2-benzisoxazol-4-yl]phenyl}-N'-(3-methylphenyl)urea;
N-{4-[3-amino-7-(4-morpholinylmethyl)-1,2-benzisoxazol-4-yl]phenyl}-N'-(2-fluoro-5-methylphenyl)urea;
N-{4-[3-amino-/-(4-morpholinylmethyl)-1,2-benzisoxazol-4-yl]phenyl}-N'-(3,5-dimethylphenyl)urea;
N-{4-[3-amino-7-(4-morpholinylmethyl)-1,2-benzisoxazol-4-yl]phenyl}-N-(3-phenoxyphenyl)urea;
N-{4-[3-amino-7-(4-morpholinylmethyl)-1,2-benzisoxazol-4-yl]phenyl}-N'-(3-bromophenyl)urea;
N-(4-{3-amino-7-[2-(4-morpholinyl)ethoxy]-1,2-benzisoxazol-4-yl}phenyl)-N'-[3-(trifluoromethyl)phenyl]urea;
N-(4-{3-amino-7-[2-(4-morpholinyl)ethoxy]-1,2-benzisoxazol-4-yl}phenyl)-N'-(2-fluoro-5-methylphenyl)urea;
N-(4-{3-amino-7-[2-(4-morpholinyl)ethoxy]-1,2-benzisoxazol-4-yl}phenyl)-N'-[2-fluoro-5-(trifluoromethyl)phenyl]urea;
N-(4-{3-amino-7-[2-(4-morpholinyl)ethoxy]-1,2-benzisoxazol-4-yl}phenyl)-N'-(3-methylphenyl)urea;
N-[4-(3-amino-1,2-benzisoxazol-4-yl)phenyl]-N'-(3,5-dimethylphenyl)urea;
N-[4-(3-amino-1,2-benzisoxazol-4-yl)phenyl]-N'-phenylurea;
N-[4-(3-amino-1,2-benzisoxazol-4-yl)phenyl]-N'-(4-methylphenyl)urea;
N-[4-(3-amino-1,2-benzisoxazol-4-yl)phenyl]-N'-(3-cyanophenyl)urea;
N-[4-(3-amino-1,2-benzisoxazol-4-yl)phenyl]-N'-[4-fluoro-3-(trifluoromethyl)phenyl]urea;
N-[4-(3-amino-1,2-benzisoxazol-4-yl)phenyl]-N'-(3-bromophenyl)urea;
N-[4-(3-amino-1,2-benzisoxazol-4-yl)phenyl]-N'-(3-chlorophenyl)urea;
N-[4-(3-amino-1,2-benzisoxazol-4-yl)phenyl]-N'-(3-ethylphenyl)urea;
N-[4-(3-amino-1,2-benzisoxazol-4-yl)phenyl]-N'-[4-(trifluoromethyl)phenyl]urea;
N-[4-(3-amino-1,2-benzisoxazol-4-yl)phenyl]-N'-(3-fluoro-4-methylphenyl)urea;
N-[4-(3-amino-1,2-benzisoxazol-4-yl)phenyl]-N'-(3-fluorophenyl)urea;
N-[4-(3-amino-1,2-benzisoxazol-4-yl)phenyl]-N'-(3,5-difluorophenyl)urea;
N-[4-(3-amino-1,2-benzisoxazol-4-yl)phenyl]-N'-(3-methoxyphenyl)urea;
N-[4-(3-amino-1,2-benzisoxazol-4-yl)phenyl]-N'-(4-methoxyphenyl)urea;
N-[4-(3-amino-1,2-benzisoxazol-4-yl)phenyl]urea;
N-[4-(3-amino-1,2-benzisoxazol-4-yl)phenyl]-N'-(3-nitrophenyl)urea;
N-[4-(3-amino-1,2-benzisoxazol-4-yl)phenyl]-N'-(4-fluorophenyl)urea;
N-[4-(3-amino-1,2-benzisoxazol-4-yl)phenyl]-N'-(2-fluorophenyl)urea;
N-[4-(3-amino-1,2-b enzisoxazol-4-yl)phenyl]-N'-(3-chloro-4-fluorophenyl)urea;
N-[4-(3-amino-1,2-benzisoxazol-4-yl)phenyl]-N'-(3-chloro-4-methoxyphenyl)urea;
N-[4-(3-amino-1,2-benzisoxazol-4-yl)phenyl]-N'-[4-(dimethylamino)phenyl]urea;
N-[4-(3-amino-1,2-benzisoxazol-4-yl)phenyl]-N'-[4-(trifluoromethoxy)phenyl]urea;
N-[4-(3-amino-1,2-benzisoxazol-4-yl)phenyl]-N'-[2-(trifluoromethoxy)phenyl]urea;
N-[4-(3-amino-1,2-benzisoxazol-4-yl)phenyl]-N'-[3,5-bis(trifluoromethyl)phenyl]urea;
N-[4-(3-amino-1,2-benzisoxazol-4-yl)phenyl]-N'-(3-chloro-4-methylphenyl)urea;
N-[4-(3-amino-7-methoxy-1,2-benzisoxazol-4-yl)phenyl]-N'-[3,5-bis(trifluoromethyl)phenyl] urea;
N-[4-(3-amino-7-methoxy-1,2-benzisoxazol-4-yl)phenyl]-N'-[4-(trifluoromethoxy)phenyl]urea;
N-[4-(3-amino-7-methoxy-1,2-benzisoxazol-4-yl)phenyl]-N'-(3-fluorophenyl)urea;
N-[4-(3-amino-7-methoxy-1,2-benzisoxazol-4-yl)phenyl]-N'-(3-methoxyphenyl)urea;
N-[4-(3-amino-7-methoxy-1,2-benzisoxazol-4-yl)phenyl]-N'-(3,5-difluorophenyl)urea;
N-[4-(3-amino-7-methoxy-1,2-b enzisoxazol-4-yl)phenyl]-N'-(4-methylphenyl)urea;
N-[4-(3-amino-7-methoxy-1,2-benzisoxazol-4-yl)phenyl]-N'-(3-bromophenyl)urea;
N-[4-(3-amino-7-methoxy-1,2-benzisoxazol-4-yl)phenyl]-N'-(3,5-dimethylphenyl)urea;
N-[4-(3-amino-7-methoxy-1,2-benzisoxazol-4-yl)phenyl]-N'-[4-(dimethylamino)phenyl]urea;
N-[4-(3-amino-7-methyl-1,2-benzisoxazol-4-yl)phenyl]-N'-(3-methylphenyl)urea;
N-[4-(3-amino-7-methyl-1,2-benzisoxazol-4-yl)phenyl]-N'-(3-chlorophenyl)urea;
N-[4-(3-amino-7-methyl-1,2-benzisoxazol-4-yl)phenyl]-N'-(2-fluoro-5-methylphenyl)urea;
N-[4-(3-amino-7-methyl-1,2-benzisoxazol-4-yl)phenyl]-N'-[2-fluoro-5-(trifluoromethyl)phenyl]urea;
N-[4-(3-amino-7-methyl-1, 2-benzisoxazol-4-yl)phenyl]-N'-[3-(trifluoromethyl)phenyl]urea;
N-[4-(3-amino-7-methyl-1,2-benzisoxazol-4-yl)phenyl]-N'-(3,5-dimethylphenyl)urea;
N-[4-(3-amino-7-methyl-1,2-benzisoxazol-4-yl)phenyl]-N'-(3-ethylphenyl)urea;
N-[4-(3-amino-7-methyl-1,2-benzisoxazol-4-yl)phenyl]-N'-(4-methylphenyl)urea;
N-[4-(3-amino-7-methyl-1,2-benzisoxazol-4-yl)phenyl]-N'-[4-(trifluoromethoxy)phenyl]urea;
N-[4-(3-amino-7-methyl-1,2-benzisoxazol-4-yl)phenyl]-N'-(3-fluoro-4-methylphenyl)urea;
N-[4-(3-amino-7-methyl-1,2-benzisoxazol-4-yl)phenyl]-N'-(3-methoxyphenyl)urea;
N-[4-(3-amino-7-methyl-1,2-benzisoxazol-4-yl)phenyl]-N'-phenylurea;
N-[4-(3-amino-7-methyl-1,2-benzisoxazol-4-yl)phenyl]-N'-[3,5-bis(trifluoromethyl)phenyl]urea;
N-[4-(3-amino-7-methyl-1,2-benzisoxazol-4-yl)phenyl]-N'-(3-bromophenyl)urea;
N-[4-(3-amino-7-methyl-1,2-benzisoxazol-4-yl)phenyl]-N'-(3-fluorophenyl)urea;
N-[4-(3-amino-7-methoxy-1,2-benzisoxazol-4-yl)phenyl]-N'-[4-fluoro-3-(trifluoromethyl)phenyl]urea;
N-[4-(3-amino-7-methoxy-1,2-benzisoxazol-4-yl)phenyl]-N'-(4-fluoro-3-methylphenyl)urea;
N-[4-(3-amino-7-fluoro-1,2-benzisoxazol-4-yl)phenyl]-N'-[3-(trifluoromethyl)phenyl]urea;
N-[4-(3-amino-7-fluoro-1,2-benzisoxazol-4-yl)phenyl]-N'-(3-chlorophenyl)urea;
N-[4-(3-amino-7-fluoro-1,2-benzisoxazol-4-yl)phenyl]-N'-[4-fluoro-3-(trifluoromethyl)phenyl]urea;
N-[4-(3-amino-7-fluoro-1,2-benzisoxazol-4-yl)phenyl]-N'-(3-methylphenyl)urea;
N-[4-(3-amino-7-fluoro-1,2-benzisoxazol-4-yl)phenyl]-N'-[2-fluoro-5-(trifluoromethyl)phenyl]urea;
N-[4-(3-amino-7-fluoro-1,2-benzisoxazol-4-yl)phenyl]-N'-(2-fluoro-5-methylphenyl)urea;
N-{4-[3-amino-7-(trifluoromethoxy)-1,2-benzisoxazol-4-yl]phenyl}-N'-[2-fluoro-5-(trifluoromethyl)phenyl]urea;
N-{4-[3-amino-7-(trifluoromethoxy)-1,2-benzisoxazol-4-yl]phenyl]-N'-[3-(trifluoromethyl)phenyl]urea;
N-{4-[3-amino-7-(trifluoromethoxy)-1,2-benzisoxazol-4-yl]phenyl}-N'-(2-fluoro-5-methylphenyl)urea;
N-{4-[3-amino-7-(trifluoromethoxy)-1,2-benzisoxazol-4-yl]phenyl}-N'-(3-chlorophenyl)urea;
N-{4-[3-amino-7-(trifluoromethoxy)-1,2-benzisoxazol-4-yl]phenyl}-N'-(3-bromophenyl)urea;
N-{4-[3-amino-7-(trifluorometlioxy)-1,2-benzisoxazol-4-yl]phenyl}-N'-[4-fluoro-3-(trifluoromethyl)phenyl]urea;
N-[4-[3-amino-1H-indazol-4-yl]phenyl]-N'-(2-fluoro-5-methoylphenyl)urea; and
N-{4-[3-amino-7-(trifluoromethoxy)-1,2-benzisoxazol-4-yl]phenyl}-N'-(4-fluoro-3-methylphenyl)urea.

The most preferred compound for use in the methods of the invention is N-[4-[3-amino-1H-indazol-4-yl]phenyl]-N'-(2-fluoro-5-methoylphenyl)urea.

Other preferred compounds for use in the methods described herein may be identified using assays described herein, the performance of which will be routine to the skilled artisan.

The RTKi may be administered via any viable delivery method or route, however, local administration is preferred. It is contemplated that all local routes to the eye may be used including topical, subconjunctival, periocular, retrobulbar, subtenon, intracameral, intravitreal, intraocular, subretinal, and suprachoroidal administration. Systemic or parenteral administration may be feasible including but not limited to intravenous, subcutaneous, and oral delivery. The most preferred method of administration will be intravitreal or subtenon injection of a solution or suspension; intravitreal or subtenon placement of a bioerodible or non-bioerodible device (implant); or by topical ocular administration of a solution or suspension. In one preferred embodiment, the compound will be administered via posterior juxtascleral administration of a solution, suspension, or gel. In another preferred embodiment, the compound will be administered via intravitreal administration of a bioerodible implant. In certain preferred aspects, the bioerodible implant will be administered intravitreally via a device such as that described in US application serial no. 60/710,046, filed August 22, 2005.

### BRIEF DESCRIPTION OF THE DRAWINGS

The following drawings form part of the present specification and are included to further demonstrate certain aspects of the present invention. The invention may be better understood by reference to these drawings in combination with the detailed description of specific embodiments presented herein.
**FIG. 1** The selective RTKi, AL-39324, inhibits preretinal neoavascularization (NV) following a single intravitreal injection in the rat model of oxygen-induced retinopathy (OIR).
**FIG. 2** The selective RTKi, AL-39324, prevents preretinal neoavascularization (NV) following oral gavage in the rat model of oxygen-induced retinopathy (OIR).
**FIG. 3** The selective RTKi, AL-39324, inhibits laser-induced choroidal neovascularization (CNV) following a single intravitreal injection in the mouse.
**FIG. 4** The selective RTKi, AL-39324, induces regression of existing laser-induced choroidal neovascularization (CNV) following a single intravitreal injection in the mouse.
**FIG. 5** Comparison of CNV lesions between AL-39324-treated groups in the mouse.
**FIG. 6** The selective RTKi, AL-39324, inhibits laser-induced choroidal neovascularization (CNV) following oral gavage in the mouse.
**FIG. 7** The selective RTKi, AL-39324, inhibits diabetes-induced retinal vascular permeability following a single intravitreal injection in the rat.
**FIG. 8** The selective RTKi, AL-39324, inhibits VEGF-induced retinal vascular permeability following a single intravitreal injection in the rat.
**FIG. 9** The selective RTKi, AL-39324 completely prevents diabetes-induced retinal vascular permeability following oral gavage in the STZ rat model.

### DETAILED DESCRIPTION PREFERRED EMBODIMENTS

According to the methods of the present invention, a composition comprising a Receptor Tyrosine Kinase inhibitor (RTKi), having a kinase inhibition profile similar to that shown in Table 1, is administered to a patient suffering from ocular neovascularization, angiogenesis, retinal edema, diabetic retinopathy, and/or retinal ischemia in order to prevent the loss of visual acuity associated with such conditions. More specifically, it is preferred that the receptor tyrosine kinase inhibitor for use in the methods of the invention block tyrosine autophosphorylation of VEGF receptor 1 (Flt-1), VEGF receptor 2 (KDR), VEGF receptor 3 (Flt-4), Tie-2, PDGFR, c-KIT, Flt-3, and CSF-1R. The present inventor has unexpectedly discovered that compounds with this unique binding profile will inhibit or prevent ocular neovascularization, retinal edema, diabetic retinopathy, and/or retinal ischemia significantly more potently and effectively than compounds currently known for such uses. More surprisingly, the compounds for use in the methods of the invention, having the preferred binding profiles described herein, cause regression of neovascularization.

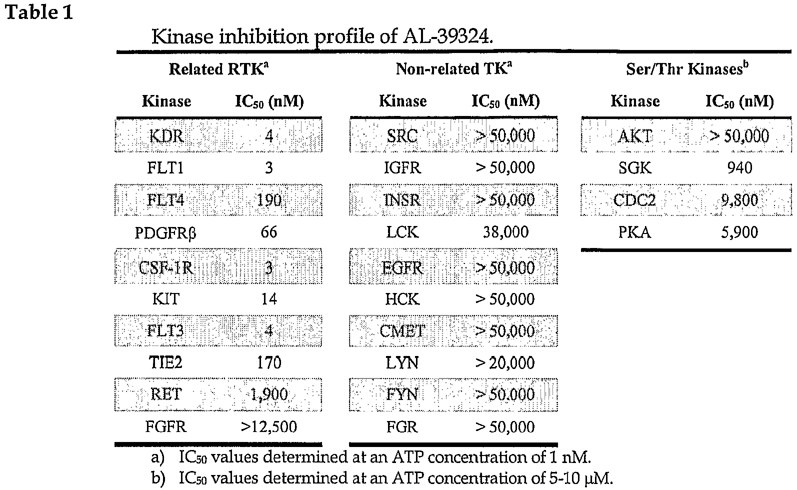

The reversible phosphorylation of proteins is one of the primary biochemical mechanisms mediating eukaryotic cell signaling. This reaction is catalyzed by protein kinases that transfer the γ phosphate group of ATP to hydroxyl groups on target proteins (Hunter, 2000). 518 such enzymes exist in the human genome, of which approximately 90 selectively catalyze the phosphorylation of tyrosine hydroxyl groups (Manning, 2002; Robinson, 2000). These human tyrosine kinases have been organized in dendrogram format based on the sequence homology of their catalytic domains (http://www.cellsignal.com/retail/). Cytosolic tyrosine kinases reside intracellularly, whereas receptor tyrosine kinases (RTKs) possess both extracellular and intracellular domains and function as membrane spanning cell surface receptors. As such, RTKs mediate the cellular responses to environmental signals and facilitate a broad range of cellular processes including proliferation, migration and survival.

Since RTKs are one of the principal components of the signaling network that transmits extracellular signals into cells, RTK dysregulation of signaling pathways is associated with a variety of human disorders including cancer and ocular disease Consequently, the utility of RTK inhibitors (RTKi), specifically antagonists of the VEGF receptor family, is well established for inhibiting angiogenesis in a variety of tissues including the eye. However, the present inventor is the first to show that simultaneous blocking of tyrosine autophosphorylation of at least one VEGF receptor along with another type of tyrosine kinase receptor will not only significantly inhibit angiogenesis (i.e. to a greater extent than has previously been seen), but also cause regression of angiogenesis.

### VEGF Receptor Family

The VEGF receptor family consists of three RTKs, KDR (kinase insert domain-containing receptor; also known as VEGFR2), FLT1 (Fms-like tyrosine kinase; also known as VEGFR1), and FLT4 (VEGFR3)(Ferrara 2003). These receptors mediate the biological function of the vascular endothelial growth factors (VEGF-A, -B, -C, -D, -E and placenta growth factor (P1GF)), a family of homodimeric glycoproteins that bind the VEGF receptors with varying affinities (Wiesmann 1997; Ferrara 1997). KDR, FLT1 and FLT4 possess three structural regions: an extracellular domain containing seven immunoglobulin-like motifs that contain the growth factor binding sites, a single transmembrane-spanning domain, and an intracellular split kinase domain that mediates the tyrosine kinase activity required for signal transduction (de Vries 1992; Terman 1992). These motifs are compared with those of structurally-related RTKs from the PDGF, FGF, RET and TIE families in the dendogram shown at http://www.cellsignal.com/retail/. For all the RTKs discussed below, ligand binding to the extracellular domain induces receptor dimerization and the autophosphorylation of specific intracellular tyrosine residues. These phosphorylated tyrosine moieties serve as docking sites for other proteins and ultimately lead to downstream signaling (Schlessinger 2000).

*VEGF, VEGFR-1 & -2:* Vascular endothelial growth factor (VEGF) binds the high affinity membrane-spanning tyrosine kinase receptors VEGFR-2 (KDR, Flak-1) and VEGFR-1 (Flt-1). Cell culture and gene knockout experiments indicate that each receptor contributes to different aspects of angiogenesis.

KDR: KDR is the major mediator of the mitogenic, angiogenic and permeability-enhancing effects of VEGF-A, hereafter referred to as VEGF. Many different cell types are able to produce VEGF, yet its biological activity is limited predominately to the vasculature by way of the endothelial cell-selective expression of KDR (Ferrara 2003; Terman 1992; Millauer 1993; Quinn 1993). Not surprisingly, the VEGF/KDR axis is a primary mediator of angiogenesis, the means by which new blood vessels are formed from preexisting vessels (Ferrara 2003; Griffioen 2000; Rak 1995). The role of this signaling pathway in developmental angiogenesis is consistent with the embryonic lethality and abnormal blood vessel formation that is observed in both VEGF- and KDR-null mice (Shalaby 1995; Carmeliet 1996).

FLT1: Despite their structural similarity, KDR and FLT1 fulfill somewhat different functions *in vivo* (Shalaby 1995; Fong 1995). FLT1 binds VEGF with high affinity, but the increase in kinase activity is not as robust as with KDR (Waltenberger 1994). FLT1 also binds VEGF-B and placental growth factor, two ligands that KDR does not bind. FLT1 is expressed on the surface of smooth muscle cells, monocytes and hematopoietic stems cells in addition to endothelial cells (Rafii 2002). Activation of FLT1 signaling results in the mobilization ot marrow-derived endothelial progenitor cells that are recruited to tumors, and potentially the diseased retina/choroid, where they contribute to new blood vessel formation (Erikson, 2002; Lyden 2001; Grant 2002; Csaky 2004).

FLT4: Despite its structural similarity to KDR and FLT1, FLT4 mediates the signaling of VEGF-C and VEGF-D, but not VEGF-A. Significantly, activation of FLT4 in the absence of KDR signaling is able to induce lymphangiogenesis and metastasis in cancer animal models (Krishnan 2003).

VEGF and its RTKs contribute to vascular morphogenesis and disease progression through their ability to mediate two predominant mechanisms: new vessel growth (vasculogenesis &/or angiogenesis) and vascular permeability (Lueng 1989; Keck 1989; Hanahan 1997; Yancopoulos 2000). Regarding the eye, VEGF is a critical developmental factor during vascular development in the posterior segment (Stone 1995). Moreover, human ocular tissues respond to a variety of stimuli, such as hypoxia, by the induction of VEGF resulting in posterior segment neovascularization and blood-retinal barrier breakdown (i.e., enhanced microvascular permeability) (Shima 1995; Hartnett 2003). VEGF and VEGFRs have been localized to neovascular tissues obtained from patients with diabetic retinopathy and exudative AMD, and are associated with increased severity of disease (Lutty 1996; Chen 1997; Witmer 2002; Kvanta 1996). Recent evidence suggests that the VEGF₁₆₅ isoform may be a primary mediator of ocular disease, however, the role of the other isoforms remains to be clearly defined (Ishida 2003; Ishida 2003).

Animal models of ocular angiogenesis and diabetic retinopathy have been used to demonstrate the critical role of VEGF signaling in posterior segment disease. Results from efficacy pharmacology studies conducted in these *in vivo* systems are used to support the utility of various treatment modalities in man. Early determination of the key role played by VEGF in pathologic ocular angiogenesis was demonstrated in a nonhuman primate model of retinal ischemia, where VEGF was spatially and temporally correlated with the NV (Miller 1994; Tolentino 1996). Moreover, intravitreal injection of VEGF produces retinal ischemia and microangiopathy in the same primate species (Tolentino 1996). Notably, intravitreal injection of a neutralizing anti-VEGF monoclonal antibody inhibited the NV displayed in this model and provided preliminary evidence that anti-VEGF therapies may have promise for human disease (Adamis 1996). Oxygen-induced retinopathy (OIR) models produce preretinal NV similar to that found in the human diseases, Retinopathy of Prematurity and PDR, and are widely used screening assays for anti-angiogenic strategies. In the rodent OIR models, retinal VEGF levels are correlated with the incidence and severity of pathology and intravitreal injection of a RTK inhibitor blocking VEGFRs provided significant reduction in retinal NV (Werdich 2004; Unsoeld 2004). The rodent and primate models of laser-induced choroidal NV are commonly used, experimental surrogates for exudative AMD and have been shown to be VEGF dependent (Shen 1998; Kwak 2000; Krzystolik 2002). Results from clinical ophthalmology studies with Macugen (an anti-VEGF aptamer, Eyetech/Pfizer) and Lucentis (a rhFab against VEGF, Genentech) have validated inhibition of VEGF signaling as a compelling ophthalmic target (Eyetech Study Group 2002; Sorbera 2003; Saishin 2003).

### Angiopoietin Receptors

Angiopoietins (Angl-4) are ligands for the Tie receptors, Tie-1 and Tie-2, a family of RTKs that are selectively expressed by vascular endothelial cells and some hematopoietic cells (Yancopoulos 2000). Tie-2-/- mice die during embryogenesis at day 9.5-10.5, where vessels are immature and lack organization (Asahara 1998). Angl and Ang2 are integrally involved in vasculogenesis and angiogenesis, acting through the Tie-2 receptor. Ang2 is upregulated in retinal endothelial cells by exposure to VEGF and hypoxia and its expression is induced during physiologic and pathologic ocular angiogenesis (Oh 1999; Hackett 2000). Signaling through Tie-2 may regulate retinal angiogenesis in concert with VEGF signaling and be a critical pathway in nonproliferative diabetic retinopathy (Sarlos 2003; Hammes 2004; Ohashi 2004; Takagi 2003). Ang2 and VEGF are co-upregulated, and Tie-2 is expressed in a variety of cell types, in choroidal neovascular membranes obtained from patients with exudative AMD (Otani 1999).

### PDGF Receptor Family

PDGFR-α & -β: The α and β isoforms of the platelet-derived growth factor (PDGF) receptors occur as homodimers or α/β heterodimers and are found most commonly on the surface of fibroblasts, smooth muscle cells, and vascular endothelial cells (Ostman 2001; Benjamin 1998). Blood vessel remodeling appears to be defined by pericyte coverage of the endothelium, which is regulated by PDGF-B and VEGF (Benjamin 1998). Tumor-associated fibroblasts are a source of growth factors, including VEGF, consequently paracrine PDGF signaling is thought to contribute to disease progression in these cancers (Ponten 1994; Skobe 1998; Fukumura 1998). PDGFR-β contributes to tumor angiogenesis through the proliferation and migration of pericytes, the peri-endothelial cells that associate with and stabilize immature blood vessels (Lindahl 1997; Hellström 1999; Reinmuth 2001; George 2001; Wang 1999). Inhibition of PDGF receptor signaling in fibroblasts and pericytes has been shown to enhance the antitumor effects of chemotherapy by regulating tumor interstitial fluid pressure (Pietras 2002). Similarly, PDGF and PDGFRs may be important in the retinal neurons and microvasculature and modulate angiogenesis in the eye (Mudhar 1993; Wilkinson 2004).

### RTKs expressed by Hematopoietic Precursor Cells

Several RTKs, including VEGFRs, CSF-1R, KIT, and FLT3, are expressed by hematopoietic precursor cells (HPCs) and may be involved in pathologic ocular angiogenesis. For example, HPCs have been shown to hone to sites of choroidal neovascularization (Espinosa 2003; Cousins 2004). However, the majority of data related to these RTKi's has been generated in oncology models. CSF-1R is encoded by the cellular homolog of the retroviral oncogene *v-fins* and is a major regulator of macrophage development (Sherr 1985). KIT is expressed by hematopoietic progenitor cells, mast cells, germ cells and by pacemaker cells in the gut (interstitial cells of Cajal) (Natali 1992; Turner 1992). It contributes to tumor progression by two general mechanisms: namely, autocrine stimulation by its ligand, stem cell factor (SCF), and through mutations that result in ligand-independent kinase activity (Heinrich 2002; Tian 1999). FLT3 is normally expressed on hematopoietic stem cells where its interaction with FLT3 ligand (FL) stimulates stem cell survival, proliferation and differentiation (Rosnet 1993; Rosnet 1996). In addition to being over-expressed in various leukemia cells (Dehmel 1996; Kiyoi 2002), FLT3 is frequently mutated in hematological malignancies with approximately one-third of patients with acute myeloid leukemia (AML) harboring activating mutations (Stirewalt 2003; Armstrong 2003; Nakao 1996; Sawyers 2002; Kottaridis 2003).

### Rationale for Multi-Targeted Receptor Tyrosine Kinase Inhibitors

Based upon the information above, protein kinases and RTKs have been targeted for designing novel pharmacologic strategies to a variety of human conditions, such as cancer and posterior segment disease (Lawrence 1998; Gschwind 2004). Consequently, numerous pharmaceutical companies have developed medicinal chemistry efforts to design both selective and multi-targeted RTK inhibitors (Traxler 2001; Murakata 2002). Highly specific inhibitors of VEGFR-2, or KDR, have been designed and demonstrate potent and efficacious inhibition of tumor-induced angiogenesis (Shaheen 2001; Boyer 2002, Bilodeau 2002; Manley 2002; and Curtin 2004). The RTKi compound, SU11248, is currently in clinical trials for cancer treatment. This compound was selected based on the performance of inhibitors with varying kinase selectivities in a transgenic mouse model of pancreatic islet cell carcinogenesis (Inoue 2002; McMahon 2002). In this model, the combination of a selective KDR inhibitor (SU5416) plus Gleevec, a PDGFR and KIT inhibitor, produced responses greater than either agent given individually (Bergers 2003). These responses included regressions of established tumors and were attributed to simultaneous inhibition of VEGF signaling in endothelial cells and PDGF signaling in pericytes, since a disruption of endothelial cell-pericyte association was observed. Significantly, no such disruption of endothelial cell-pericyte junctions was seen in the non-tumor vasculature from these animals.

Related to ophthalmic indications, Campochiaro *et al.* demonstrated that oral administration of a RTKi selective for VEGFRs, PDGFRs, and PKC (*i.e*., PKC-412), inhibited both preretinal and choroidal NV in mice (Seo 1999). Using oral administration of RTKi's with different selectivity profiles, Campochiaro demonstrated that blockade of VEGFR-2 was sufficient to completely prevent retinal NV, but did not affect adult, quiescent retinal capillaries (Ozaki 2000). Following these preclinical results, PKC-412 was assessed in human patients with diabetic macular edema (Campochiaro 2004). Although pilot results suggested a reduction in macular edema and an improvement in visual acuity, concerns related to liver toxicity halted the clinical trials. More recently, intravitreal injection of an RTKi that blocks VEGFR-2, IGF-1R, FGFR-1, and EGFR provided a modest reduction (25%) in the median retinopathy score in the mouse OIR model (Unsoeld 2004). None of the compounds tested in the above studies have the particular receptor binding profile of the compounds useful in the methods of the present invention. The present inventor has demonstrated for the first time that compounds having the receptor binding profile described herein exhibit unique and unexpected results with respect to inhibiting neovascularization and/or angiogenesis.

The present inventor has discovered for the first time that, for effective inhibition of neovascularization, it is important that the therapeutic compound have activity at multiple receptors as described herein. The RTKi's claimed herein provide reproducible efficacy against pathologic ocular angiogenesis and vascular permeability following local or systemic therapy. Furthermore, the RTKi's described herein for use in the methods of the invention cause regression of ocular neovascularization and/or angiogenesis. Unexpectedly, the RTKi's claimed here provide several novel advantages as related to ophthalmic use versus other publicly disclosed compounds.

### Identification and Kinase Selectivity of Preferred Compounds

The high homology in secondary structure of certain RTKs, such as VEGF and PDGF receptors, catalytic domains suggests the possibility of identifying compounds that inhibit multiple family members. The preferred compounds for use in the methods of the present invention have such a profile. Assays for determining receptor binding activity of test compounds that are well known to the skilled artisan may be used to identify additional potential compounds for use in the methods of the present invention.

The models described in the examples below can be used to identify additional effective compounds for potential use in the methods of the invention, or to select preferred compounds from those identified via receptor binding assays that are well known to the skilled artisan. For example, a test compound may be evaluated in the rat OIR model described in Example 1, the mouse laser model described in Example 3, the rat VEGF model described in Example 6, and the diabetic rat model described in Example 7. Potential RTKi's (test compounds) for use in the methods of the invention, would preferably provide:
- >75% inhibition of preretinal NV in the rat OIR model following a single intravitreal injection of ≤3% solution or suspension, or oral gavage with a solution or suspension ≤30mg/kg/d.
- >70% inhibition of choroidal NV in the mouse laser model following a single intravitreal injection of ≤3% solution or suspension, or oral gavage with a solution or suspension <30mg/kg/d.
- >50% inhibition of retinal vascular permeability in rat VEGF model following a single intravitreal injection of ≤3% solution or suspension, or oral gavage with a solution or suspension <30mg/kg/d.
- >75% inhibition of retinal vascular permeability in the STZ-induced diabetic rat model following a single intravitreal injection of ≤3% solution or suspension, or oral gavage with a solution or suspension <30mg/kg/d.

Compounds that are able to achieve activity in the categories described above would be preferred agents with potential clinical utility. More preferred agents would also exhibit >25% regression of choroidal NV in the mouse laser model following a single intravitreal injection of ≤3% solution or suspension, or 50% regression with oral gavage with a solution or suspension <30mg/kg/d.

The most preferred compound for use in the present invention, AL-39324 (N-[4-[3-amino-1H-indazol-4-yl]phenyl]-N'-(2-fluoro-5-methoylphenyl)urea), is a potent, ATP-competitive inhibitor of all members of the VEGF and PDGF receptor tyrosine kinases but lacks significant inhibition of other tyrosine and serine/threonine kinases. Its kinase inhibition profile is shown in Table 1. It was identified based on its activity in primary VEGF/PDGF receptor enzyme assays, growth factor-stimulated cellular assays and a mouse model of estradiol-induced uterine edema, all assays that are well known to the skilled artisan. Several series of novel, ATP-competitive multi-targeted RTK inhibitors were identified using this testing strategy, including the urea-substituted aminoindazoles of which AL-39324 is a member.

Preferred RTKi compounds for use in the methods of the present invention are potent, competitive inhibitors of the ATP binding site for a select group of RTKs. That is, preferred agents simultaneously block tyrosine autophosphorylation of VEGF receptor 1 (Flt-1), VEGF receptor 2 (KDR), VEGF receptor 3 (Flt-4), TIE-2, PDGFR, c-KIT, FLT-3, and CSF-1R activity at low nM concentrations. Preferably, compounds for use in the methods of the invention exhibit an IC₅₀ range between 0.1 nM and 250 nM for each of these receptors. More preferred compounds exhibit an IC₅₀ range between 0.1 nM and 100 nM for at least six of these receptors. Most preferred compounds possess an IC₅₀ range between 0.1 nM and 10nM for at least four of these receptors.

In other embodiments, the RTKi for use in the methods of the invention will simultaneously block tyrosine autophosphorylation of (a) KDR, Flt-1, PDGFR, and Tie-2; (b) KDR, Flt-1, and Tie-2; (c) KDR, Flt-1, and PDGFR; (d) KDR, Tie-2, and PDGFR; (e) KDR and Tie-2; and (f) KDR and PDGFR. It will be understood that the above described preferred binding profiles are presented in no particular order of preference but simply represent additional preferred binding profiles for the compounds useful in the methods of the invention.

The present inventor has shown that RTKi's with the binding profile described herein reproducibly inhibit and regress retinal (Examples 1-2) and choroidal neovascularization (Examples 3-5), as well as block VEGF-enhanced (Example 6) and diabetes-induced (Example 7) retinal vascular permeability. No previously known RTKi compounds have exhibited the preferred binding profile or exhibited complete inhibition plus regression of retinal and choroidal neovascularization and blocked VEGF- and diabetes-enhanced retinal vascular permeability as potently or effectively.

The most preferred compound for use in the methods of the present invention is N-[4-[3-amino-1H-indazol-4-yl)phenyl]-N'-(2-fluoro-5-methylphenyl)urea (also referred to herein as AL-39324), having the following structure:

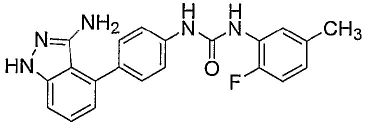

As demonstrated above, the RTKi's claimed within this invention provide several distinct and novel advantages against other published tyrosine kinase inhibitors when used for the eyes: 1) highly potent and efficacious inhibition of retinal and choroidal neovascularization, 2) regression of established CNV, 3) pronounced inhibition of VEGF- and diabetes-induced retinal vascular permeability, and 4) intraocular tolerability (Example 8). The novel RTK selectivity profile provided by preferred RTKi's for use in the methods of the invention likely accounts for their distinguished activity in comparison with previously described RTKi's and other classes of compounds. The selectivity profile of these compounds, coupled with their physicochemical properties, make them candidates for novel delivery through local administration. Overall, these characteristics provide the preferred compounds with discriminating advantages in both efficacy and safety during the treatment of the most common causes of acquired blindness.

Certain RTKi's are known to possess antiangiogenic activity and have been claimed for ophthalmic and non-ophthalmic indications. For example, a variety of U.S. and international patents/patent applications claim the use of inhibitors of protein tyrosine kinases as antiangiogenic agents: U.S. Patent Nos. 6,177,401 B1; 5,773,459; 6,448,277 B2; 6,765,012 B2; U.S. Patent Applications Nos. 2004/0002501 A1; PCT Patent Nos. WO 01/85691 A1; PCT Patent Application Nos. WO 00/67738; 03/22852 A2; 03/068228; 03013439/JP; 03/080625; and European Patent Application Nos. EP 02787595 2002. None of these references suggest that it is preferable to treat ophthalmic indications using RTKi's having the particular preferred binding profiles of the compounds described herein.

The preferred RTKi's for use in the methods of the present invention are compounds described in U.S. application 20050020603, filed May 10, 2004, and PCT application no. PCT/US04/16166, filed May 21, 2004, both based upon provisional application no. 60/472,810 filed May 22, 2003.

The RTKi for use in the methods of the invention may be administered via any viable delivery method or route, however, local administration is preferred. It is contemplated that all local routes to the eye may be used including topical, subconjunctival, periocular, retrobulbar, subtenon, intracameral, intravitreal, intraocular, subretinal, and suprachoroidal administration. Systemic or parenteral administration may be feasible including but not limited to intravenous, subcutaneous, and oral delivery. The most preferred method of administration will be intravitreal or subtenon injection of solutions or suspensions, or intravitreal or subtenon placement of bioerodible or non-bioerodible devices, or by topical ocular administration of solutions or suspensions, or posterior juxtascleral administration of a gel formulation. Another preferred method of delivery is intravitreal administration of a bioerodible implant administered through a device such as that described in US application serial number 60/710,046, filed August 22, 2005.

In general, the doses used for the above described purposes will vary, but will be in an effective amount to inhibit or cause regression of neovascularization or angiogenesis. In other aspects, the doses will be in an effective amount to prevent or treat AMD, DR, sequela associated with retinal ischemia, and macular and/or retinal edema. As used herein, the term "pharmaceutically effective amount" refers to an amount of one or more RTKi which will effectively treat AMD, DR, and/or retinal edema, or inhibit or cause regression of neovascularization or angiogenesis, in a human patient. The doses used for any of the above-described purposes will generally be from about 0.01 to about 100 milligrams per kilogram of body weight (mg/kg), administered one to four times per day. When the compositions are dosed topically, they will generally be in a concentration range of from 0.001 to about 5% w/v, with 1-2 drops administered 1-4 times per day. For intravitreal, posterior juxtascleral, subTenon, or other type of local delivery, the compounds will generally be in a concentration range of from 0.001 to about 10% w/v. If administered via an implant, the compounds will generally be in a concentration range of from 0.001 to about 40% w/v.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### Example 1

### Prevention of preretinal neovascularization following intravitreal delivery of the receptor kinase tyrosine inhibitor (RTKi), AL-39324, in the Rat Model of Oxygen-induced Retinopathy

**METHODS:** Pregnant Sprague-Dawley rats were received at 14 days gestation and subsequently gave birth on Day 22 ± 1 of gestation. Immediately following parturition, pups were pooled and randomized into separate litters (n=17 pups/litter), placed into separate shoebox cages inside oxygen delivery chamber, and subjected to an oxygen-exposure profile from Day 0-14 postpartum. Litters were then placed into room air from Day 14/0 through Day 14/6 (days 14-20 postpartum). Additionally on Day 14/0, each pup was randomly assigned as an oxygen-exposed control or into various treatment groups. For those randomized into an injection treatment group: one eye received a 5 µl intravitreal injection of 0.1%, 0.3%, 0.6%, or 1% AL-39324 and the contralateral eye received a 5 µl intravitreal injection of vehicle. At Day 14/6 (20 days postpartum), all animals in both studies were euthanized.

Immediately following euthanasia, retinas from all rat pups were harvested, fixed in 10% neutral buffered formalin for 24 hours, subjected to ADPase staining, and fixed onto slides as whole mounts. Digital images were acquired from each retinal flat mount that was adequately prepared. Computerized image analysis was used to obtain a NV clockhour score from each readable sample. Each clockhour out of 12 total per retina was assessed for the presence or absence of preretinal NV. Statistical comparisons using median scores for NV clockhours from each treatment group were utilized in nonparametric analyses. Each noninjected pup represented one NV score by talking the average value ot both eyes and was used in comparisons against each dosage group. Because the pups were randomly assigned and no difference was observed between oxygen-exposed control pups from all litters, the NV scores were combined for all treatment groups. P ≤ 0.05 was considered statistically significant.

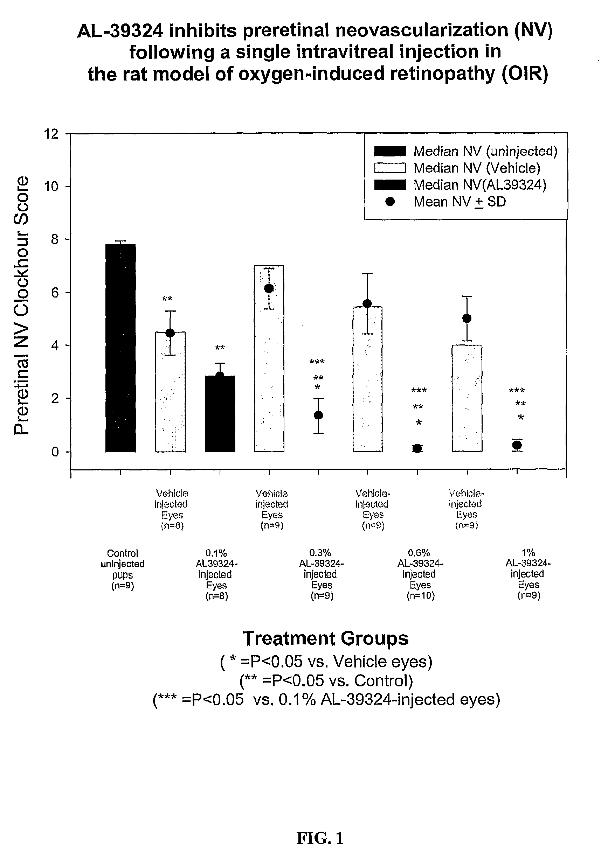

**RESULTS:** Local administration of AL-39324 provided potent anti-angiogenic efficacy against preretinal neovascularization, where 100% inhibition of preretinal NV was observed between 0.3%-1% suspensions. An overall statistical difference was demonstrated between treatment groups (Kruskal-Wallis one-way ANOVA test: *P*<0.001) (FIG. 1). Eyes treated with 0.3-1% AL-39324 exhibited significant inhibition of preretinal NV as compared to vehicle-injected injected and control, noninjected eyes (Table 2). Efficacy was not observed in 0.1% treated eyes.

**Table 2**

| Treatment | % Inhibition (vs. vehicle-inj ected eye) | *P* value | Median NV | NV Range | Median NV (Vehicle) | NV Range (Vehicle) |
|---|---|---|---|---|---|---|
| Untreated control | | | 7.8 | 3.2-10.9 | | |
| 0.1% AL-39324 | 37 | 0.161 | 2.835 | 1.1-5.3 | 4.5 | 1.1-8.4 |
| 0.3% AL-39324 | 100 | 0.002 | 0 | 0-5 | 7 | 2-8.72 |
| 0.6% AL-39324 | 100 | <0.001 | 0 | 0-1.1 | 5.45 | 2-10.9 |
| 1% AL-39324 | 100 | <0.001 | 0 | 0-2 | 4 | 1-8 |

### Example 2

### Systemic administration of AL-39324 (RTKi) potently prevents preretinal neovascularization in the Rat OIR Model.

**METHODS:** Pregnant Sprague-Dawley rats were received at 14 days gestation and subsequently gave birth on Day 22 ± 1 of gestation. Immediately following parturition, pups were pooled and randomized into separate litters (n=17 pups/litter), placed into separate shoebox cages inside oxygen delivery chamber, and subjected to an oxygen-exposure profile from Day 0 to Day 14 postpartum. Litters were then placed into room air from Day 14/0 through Day 14/6 (days 14-20 postpartum). Additionally on Day 14/0, each pup was randomly assigned as oxygen-exposed controls, vehicle treated, or drug-treated at 1.5, 5, 10 mg/kg, p.o., BID. At Day 14/6 (20 days postpartum), all animals in both studies were euthanized and retina whole mounts were prepared as described in Example 1 above.

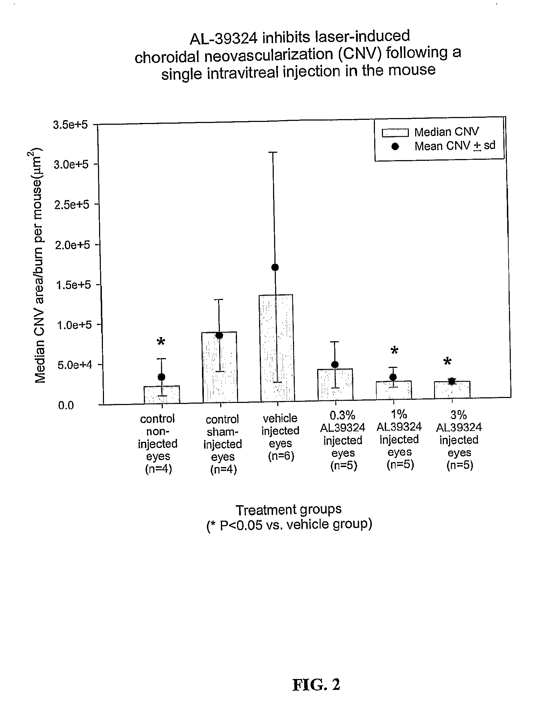

**RESULTS:** Systemic administration of the RTKi, AL-39324, provided potent efficacy in the rat OIR model, where 20mg/kg/day p.o. provided complete inhibition of preretinal NV. An overall statistical difference was demonstrated between treatment groups and non-treated controls (Kruskal-Wallis one-way ANOVA test: *P*<0.001) (FIG. 2, Table 3). Pups receiving 10 and 20 mg/kg/day p.o. demonstrated significant inhibition of preretinal NV as compared to vehicle-treated pups, where the highest dose provided complete inhibition (Mann-Whitney rank sum test: *P*= 0.005 and *P*< 0.001). Pups receiving 3 mg/kg/day p.o. did not have a significant decrease in NV.

**Table 3**

| Treatment | % Inhibition (vs vehicle-inj ected eye) | *P* value | Median NV | NV Range |
|---|---|---|---|---|
| Untreated control | | 0.427 | 5.885 | 2.5-10.5 |
| PEG 400 (vehicle) | | | 7.4 | 3.5-9.5 |
| 3 mg/kg AL-39324 in PEG 400 | -1.4 | 0.91 | 7.5 | 3.8-9.8 |
| 10 mg/kg AL-39324in PEG 400 | 86.4 | 0.001 | 1.105 | 0-8 |
| 20 mg/kg AL-39324 in PEG 400 | 100 | <0.001 | 0 | 0 |

### Example 3

### Prevention of laser-induced choroidal neovascularization (CNV) following a intravitreal delivery of the receptor kinase tyrosine inhibitor (RTKi), AL-39324, in the mouse.

Methods. CNV was generated by laser-induced rupture of Bruch's membrane. Briefly, 4 to 5 week old male C57BL/6J mice were anesthetized using intraperitoneal administration of ketamine hydrochloride (100mg/kg) and xylazine (5mg/kg) and the pupils of both eyes dilated with topical ocular instillation of 1% tropicamide and 2.5% Mydfin®. One drop of topical cellulose (Gonioscopic®) was used to lubricate the cornea. A hand-held cover slip was applied to the cornea and used as a contact lens to aid visualization of the fundus. Three to four retinal bums were placed in randomly assigned eye (right or left eye for each mouse) using the Alcon 532nm EyeLite laser with a slit lamp delivery system. The laser burns were used to generate a rupture in Bruch's membrane, which was indicated ophthalmoscopically by the formation of a bubble under the retina. Only mice with laser bums that produced three bubbles per eye were included in the study. Bums were typically placed at the 3, 6, 9 or 12 o'clock positions in the posterior pole of the retina, avoiding the branch retinal arteries and veins.

Each mouse was randomly assigned into one of the following treatment groups: noninjected controls, sham-injected controls, vehicle-injected mice, or one of three RTKi-injected groups. Control mice received laser photocoagulation in both eyes, where one eye received a sham injection, i.e. a pars plana needle puncture. For intravitreal-injected animals, one laser-treated eye received a 5ul intravitreal injection of 0%, 0.3%, 1%, or 3% AL-39324. The intravitreal injection was performed immediately after laser photocoagulation. At 14 days post-laser, all mice were anesthetized and systemically perfused with fluorescein-labeled dextran. Eyes were then harvested and prepared as choroidal flat mounts with the RPE side oriented towards the observer. All choroidal flat mounts were examined using a fluorescent microscope. Digital images of the CNV were captured, where the CNV was identified as areas of hyperfluorescence within the pigmented background. Computerized image analysis was used to delineate and measure the two dimensional area of the hyperfluorescent CNV per lesion (um²) for the outcome measurement. The median CNV area/burn per mouse per treatment group or the mean CNV area/burn per treatment group was used for statistical analysis depending on the normality of data distribution; P ≤ 0.05 was considered significant.

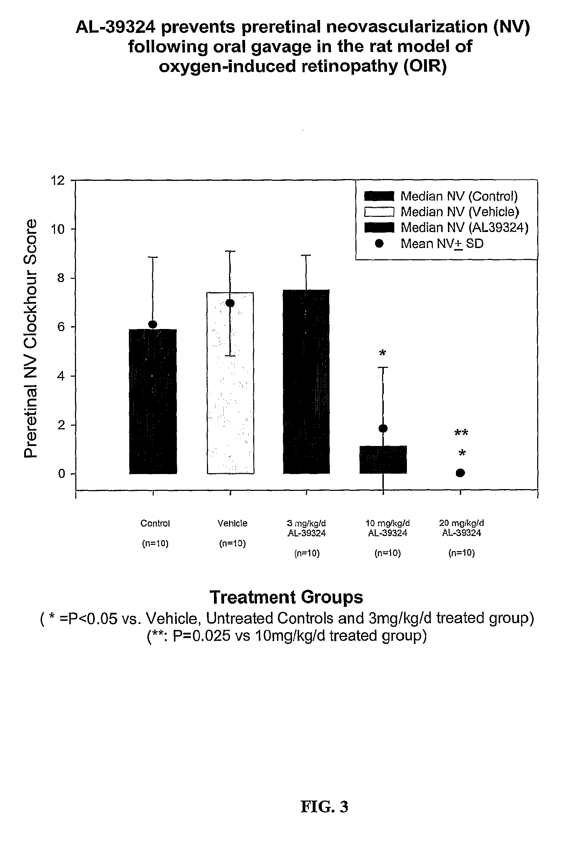

**Results.** Local administration of the RTKi, AL-39324, provided potent antiangiogenic efficacy in a mouse model of laser-induced CNV. An overall significant difference between treatment groups was established with a Kruskal-Wallis one way ANOVA (*P* = 0.015) (FIG. 3). Moreover, eyes injected with 1% AL-39234 (↓84.1%) and 3%-39234 (↓ 83.0%) showed significant inhibition of CNV as compared to vehicle-injected eyes (Mann-Whitney rank sum tests; *P*=0.004, and *P*=0.017, respectively). A marginal statistical difference was found between eyes injected with 0.3% AL-39234 and vehicle injected eyes (P=0.082).

The median and mean ± s.d. CNV area/ bum per mouse in control groups with no injection was 21721um² and 32612 ± 23131um² (n=4 mice), and with sham injection was 87854um² and 83524 ± 45144um²(n=4 mice). The median and mean ± s.d. CNV area/bum per mouse in vehicle-treated mice was 133014um² and 167330±143201 um² (n=6 mice). The median/mean ± s.d. in the 0.3%, 1% and 3% AL-39324 treated groups were 38891um² and 44283± 28886um² (n=5 mice); 21122um² and 21036±3100um² (n=5 mice); 22665um² and 27288±12109um² (n=5 mice), respectively.

### Example 4

### Intravitreal delivery of the RTKi, AL-39324, induces regression of existing laser-induced choroidal neovascularization (CNV) in the mouse

**METHODS:** CNV was generated by laser-induced rupture of Bruch's membrane as described above in Example 3. Each mouse was randomly assigned to one of the following treatment groups: noninjected controls, sham-injected controls, vehicle-injected mice, AL-39324 injected groups. Control mice received laser photocoagulation in both eyes, where one eye received a sham injection, i.e. a pars plana needle puncture. For intravitreal-injected animals, one laser-treated eye received a 5µl intravitreal injection of 0%, 1% or 3%AL-39324 or 2µl 1% AL-39324. All mice received laser photocagulation at day 0. For mice randomized to an injection group, a single intravitreal injection was performed at 7 days post-laser. Also at 7 days post-laser, several mice with no-injection were euthanized and their eyes used for controls. At 14 days post-laser, all remaining mice were euthanized and systemically perfused with fluorescein-labeled dextran. Eyes were then harvested and prepared as choroidal flat mounts with the RPE side oriented towards the observer. Choroidal flat mounts were analyzed as described above in Example 3.

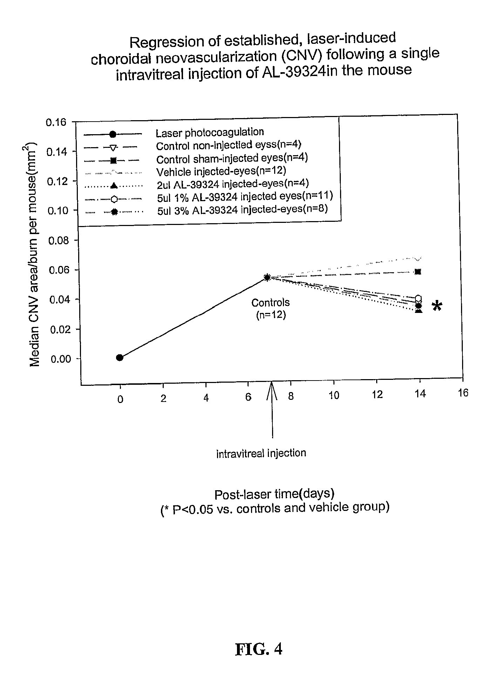

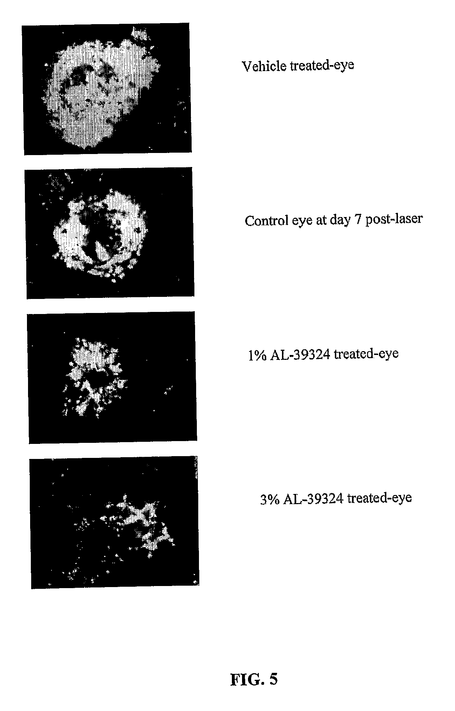

**RESULTS:** Local administration of the RTKi, AL-39324, caused regression of existing laser-induced CNV in the adult mouse. An overall significant difference between treatment groups was established with a Kruskal-Wallis one way ANOVA (*P* = 0.002) (FIG. 4). By 14 days following laser rupture of Bruch's membrane, the median CNV area in eyes injected with 2µl 1% AL-39324 (↓45.4%), 5µl 1% AL-39324 (↓29.7%), and 5µl 3% AL-39324 (↓41.0%) was significantly reduced when compared to the amount of CNV present at 7 days post-laser (Mann-Whitney rank sum tests; *P*=0.025, P=0.039 and P=0.012, respectively). Eyes injected with 2µl 1% AL-39234 (↓55.9%), 5µl 1% AL-39234 (↓43.7%), and 3%-39234 (↓ 52.3%) showed significant inhibition of CNV as compared to vehicle-injected eyes at day 14 post-laser (Mann-Whitney rank sum tests; *P*=0.009, *P*=0.006, and 0.001, respectively). A gross reduction in CNV development was observed as a decrease in the hyperfluorescent area at the site of laser photocoagulation in 1% or 3% AL-39324-injected eyes as compared to 1) control eyes at day 7 post-laser and 2) vehicle-injected eyes at day 14 post-laser (FIG. 5).

**Table 4**

| | **Median CNV(µm)** | **Mean CNV(µm)** | **SE** | **N (mice)** |
|---|---|---|---|---|
| Control at day 7 | 51808 | 54452 | 5385 | 12 |
| Non-inj ected control at day 14 | 32881 | 34589 | 8413 | 4 |
| Sham-injected control at day 14 | 54078 | 48594 | 8614 | 4 |
| Vehicle | 64067 | 65932 | 5833 | 12 |
| 1%AL-39324(2µl) | 28268 | 30959 | 7287 | 4 |
| 1% AL-39324(5µl) | 36429 | 39178 | 5861 | 11 |
| 3% AL-39324(5µl) | 30560 | 35174 | 4110 | 8 |

### Example 5

### Systemic administration of the RTKi, AL-39324, provides dose-dependent inhibition and regression of laser-induced choroidal neovascularization (CNV) in the mouse.

**METHODS:** CNV was generated by laser-induced rupture of Bruch's membrane as described in Example 3 above. Mice were randomly assigned as oral gavage groups receiving 0, 3, 10, and 20mg/kg/day AL-39324. The mice received an oral gavage of 0, 1.5, 5, or 10mg/kg twice per day and for 14 days post-laser. For the regression or intervention paradigm, mice were randomly assigned to groups receiving 0, 1.5, 5, or 10 mg/kg AL-39324 p.o. BID, (0, 3, 10, or 20 mg/kg/day) at day 7 after laser photocoagulation. Oral gavage dosing was continued twice per day for 14 days post-laser. Several mice were euthanized at day 7 post-laser and used for controls. At 14 days post-laser, all mice were anesthetized and systemically perfused with fluorescein-labeled dextran. Eyes were then harvested and prepared as choroidal flat mounts as described in Example 3 above.

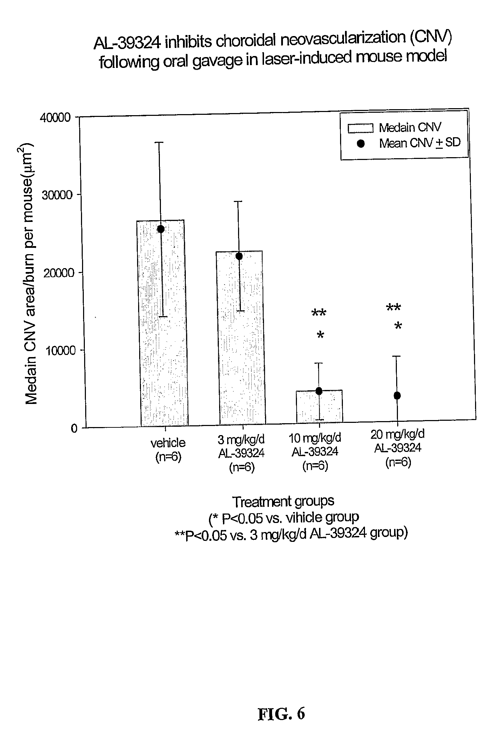

**RESULTS.** Systemic administration of the lead RTKi, AL-39324, provided potent and highly efficacious inhibition of laser-induced CNV, where mice treated 20mg/kg/day showed complete inhibition of CNV development and significant regression of established CNV. In the prevention paradigm, an overall significant difference between treatment groups was established with a Kruskal-Wallis one way ANOVA (*P* <0.001) (FIG. 6, Table 5a). Moreover, systemic delivery of 20 mg/kg/d AL-39324 provided complete inhibition of CNV (P<0.009) and the mice treated with 10mg/kg/day showed an 84.3% inhibition of CNV(P<0.002). Mice treated with 3mg/kg/day exhibited no significant inhibition (P<0.589), as compared to vehicle-injected eyes (Mann-Whitney rank sum tests).

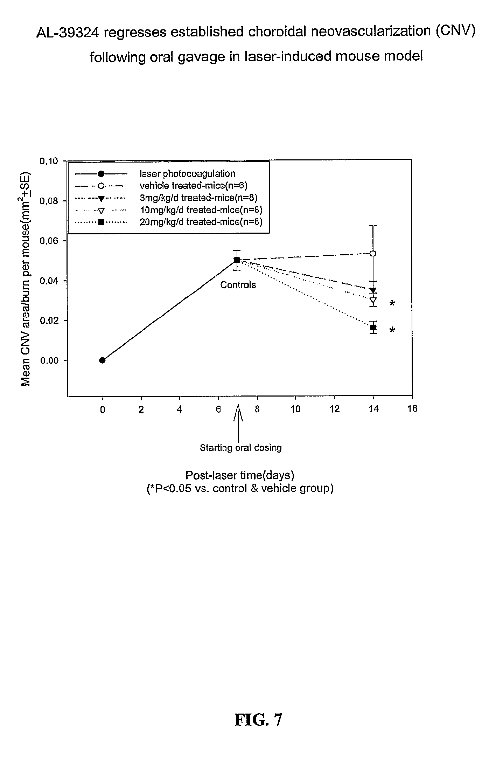

In the regression paradigm, an overall significant difference between treatment groups was established with a Kruskal-Wallis one-way ANOVA (*P*<0.001) (Figure 7 & Table 5b). Mice treated with 20 mg/kg/day and 10 mg/kg/day exhibited significant regression of existing CNV by 68.0% and 41.8%, respectively, as compared to nontreated controls (Mann-Whitney Rank Sum Test, *P*<0.002 and *P*<0.011, respectively). Mice treated with 3 mg/kg/day did not show a significant regression of existing CNV(Mann-Whitney Rank Sum Test, *P*>0.065). No significant difference was found between the control and vehicle treated-groups (Mann-Whitney Rank Sum Test, *P*=0.792).

**Table 5a**

| | Median CNV(µm²) | Mean CNV(µm²) | SD | Mice number |
|---|---|---|---|---|
| vehicle | 26417 | 25316 | 11196 | 6 |
| 3mg/kg/day AL-39324 | 22317 | 21670 | 7012 | 6 |
| 10mg/kg/day AL-39324 | 4137 | 4046 | 3625 | 6 |
| [20mg/kg/day AL-39324 | 0 | 3266 | 5079 | 6 |

**Table 5b**

| Treatment | Median CNV(µm²) | Mean CNV (µm²) | SD | Mice number |
|---|---|---|---|---|
| control | 147055 | 49665 | (11183 | 5 |
| vehicle | 41362 | 52974 | 33403 | 6 |
| 3 mg/kg/day AL-39324 | 33967 | 35442 | 11807 | 8 |
| 10 mg/kg/day AL-39324 | 27389 | 29773 | 9514 | 8 |
| 20 mg/kg/day AL-39324 | 15036 | 15706 | 8301 | 8 |

### Example 6

### Intravitreal delivery of the RTKi, AL-39324, inhibits VEGF-induced retinal vascular permeability in the rat

**METHODS:** Adult Sprague-Dawley rats were anesthetized with intramuscular ketamine/ xylazine and their pupils dilated with topical cycloplegics. Rats were randomly assigned to intravitreal injection groups of 0% 0.3%, 1.0%, and 3.0% AL-39324 and a positive control. Ten µl of each compound was intravitreally injected in each treatment eye (n=6 eyes per group). Three days following first intravitreal injection, all animals received an intravitreal injection of 10 µl 400 ng hr VEGF in both eyes. Twenty-four hours post-injection of VEGF, intravenous infusion of 3% Evans blue dye was performed in all animals, where 50mg/kg of Evans blue dye was injected via the lateral tail vein during general anesthesia. After the dye had circulated for 90 minutes, the rats were euthanized. The rats were then systemically perfused with balanced salt solution, and then both eyes of each rat were immediately enucleated and the retinas harvested using a surgical microscope. After measurement of the retinal wet weight, the Evans blue dye was extracted by placing the retina in a 0.2 ml formamide (Sigma) and then the homogenized and ultracentrifuged. Blood samples were centrifuged and the plasma diluted 100 fold in formamide. For both retina and plasma samples, 60 µl of supernatant was used to measure the Evans blue dye absorbance (ABS) with at 620/740 nm. The blood-retinal barrier breakdown and subsequent retinal vascular permeability as measured by dye absorbance were calculated as means +/-s.e.m. of net ABS/wet weight/plasma ABS. A two-tailed Student's t-test was used for pair wise comparisons between OS and OD eyes in each group. One way ANOVA was used to determine an overall difference between treatment means, where *P* ≤ 0.05 was considered significant.

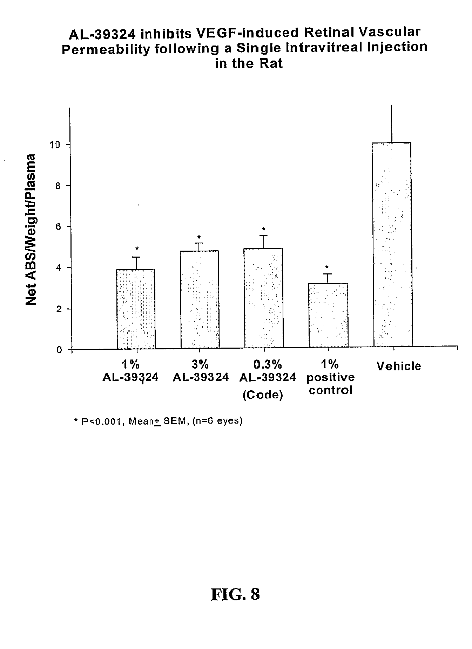

**RESULTS.** A single intravitreal injection of AL-39324 provided potent and efficacious inhibition of VEGF-induced retinal vascular permeability in the rat (FIG. 8). An overall statistical difference was demonstrated between treatment groups and vehicle controls (Student-Newman-Keuls one-way AVOVA test: P<0.001). Retinal vascular permeability was significantly decreased in eyes treated with AL-39324 as compared to vehicle-injected eyes: 0.3% AL-39324 (↓50%), 1.0% AL-39324 (↓61%), 3% AL-39324 (↓53%), and positive control (↓69%), respectively.

The mean ABS ±s.e.m. in vehicle control group was 9.93 ± 1.82. In drug treated group of 0.3% AL-39324 was 4.84 ± 0.64; in 1.0% AL-39324 group was 3.87 ± 0.62; in 3.0% AL-39324 group was 4.75 ± 0.40 and in the positive control group was 3.11 ± 0.46. There was no significant difference between drug treated groups.

### Example 7

### Intravitreal delivery of the RTKi, AL-39324, inhibits VEGF-induced retinal vascular permeability in the rat

**METHODS: D**iabetes was induced in male Long-Evans rats with 65 mg/kg streptozotocin (STZ) after an overnight fast. Upon confirmation of diabetes (blood glucose > 250 mg/dl), treatment was initiated by oral gavage. Non-diabetic (NDM) and diabetic (DM) rats received oral gavage of either vehicle or AL-39324 at 1.5 or 5 mg/kg/d BID. After 2 weeks, jugular vein catheters were implanted 1 day prior to experimentation for the infusion of indicator dye. Retinal vascular permeability, RVP, was measured using Evan's blue albumin permeation (45 mg/kg) after a 2 hour circulation period.

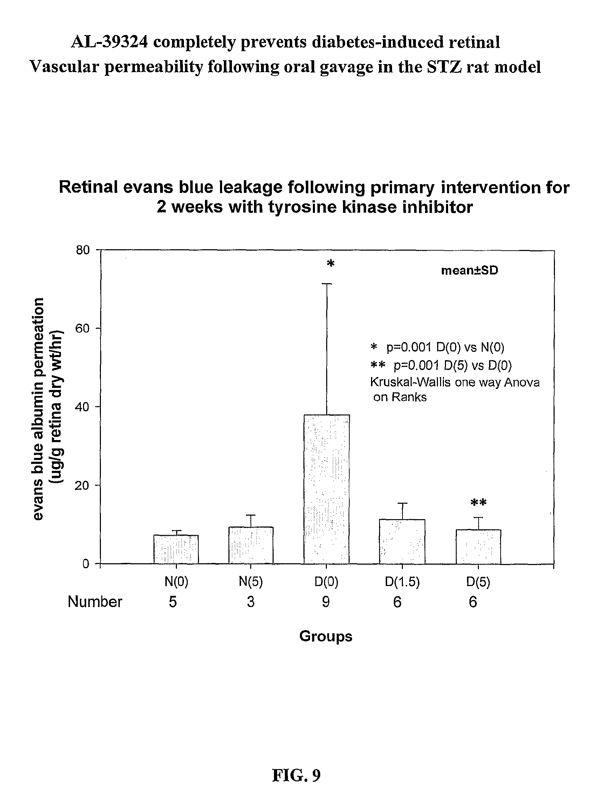

**RESULTS:** Treatment with the oral RTKi was well tolerated by both NDM and DM groups with no observed systemic or ERG side effects. Blood glucose levels and body weights were not different between DM control and DM treatment groups. Diabetes increased RVP (38.1+33.4 µl/g/hr, n=9) as compared with NDM control (7.3±2.5 µl/g/hr, n=5, p<0.001). RVP was significantly reduced in DM animals treated with AL-39324 at 1.5mg/kg/d (11.4±4.1 µl/g/hr, n=6, p<0.05) and at 5 mg/kg/d (8.9±3.1 µl/g/hr, n=7, p<0.01) as compared to DM control (Fig. 9). RVP was unchanged in NDM treated at 5 mg/kg/d.

### Example 8

### Preliminary intraocular safety study using a single intravitreal injection of AL-39324 in the adult rat

A pilot intraocular safety study (non-GLP) was completed using a single intravitreal injection of 0, 0.1, and 1.0% AL-39324 in adult rats. Outcome measures were followed up to 1 month postinjection and involved clinical (fundus photography & indirect ophthalmoscopy), functional (electroretinography), and morphologic (histopathology) methods. No significant adverse events were observed in animals treated with AL-39324 as compared to vehicle-injected controls.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. More specifically, it will be apparent that certain agents which are both chemically and structurally related may be substituted for the agents described herein to achieve similar results. All such substitutions and modifications apparent to those skilled in the art are deemed to be within the spirit, scope and concept of the invention as defined by the appended claims.

### References

The following references, to the extent that they provide exemplary procedural or other details supplementary to those set forth herein, are specifically incorporated herein by reference.
http://www.cellsignal.com/retail/
Adamis AP, Shima DT, Tolentino MJ, et al. Inhibition of vascular endothelial growth factor prevents retinal ischemia-associated iris neovascularization in a nonhuman primate. Arch Ophthalmol. 1996;114:66-71.
Armstrong SA, Kung AL, Mabon ME, et al. Inhibition of FLT3 in MLL. Validation of a therapeutic target identified by gene expression based classification. Canc Cell. 2003;3:173-83.
Asahara T, Chen D, Takahashi T, et al. Tie2 receptor ligands, angiopoietin-1 and angiopoietin-2, modulate VEGF-induced postnatal neovascularization. Circ Res. 1998;83:233-40.
Benjamin Le, Hemo I, Keshet E. A plasticity window for blood vessel remodelling is defined by pericyte coverage of the preformed endothelial network and is regulated by PDGF-B and VEGF. Development. 1998;125:1591-8.
Bergers G, Song S, Meyer-Morse N, Bergsland E, Hanahan D. Benefits of targeting both pericytes and endothelial cells in the tumor vasculature with kinase inhibitors. J Clin Inv. 2003;111:1287-95.
Bilodeau MT, Fraley ME, Hartman GD. Kinase insert domain-containing receptor kinase inhibitors as anti-angiogenic agents. Expert Opin Investig Drugs. 2002;11(6):737-4.5.
Blume-Jensen P, Hunter T. Oncogenic kinase signalling. Nature. 2001;411:355-65.
Boyer SJ. Small molecule inhibitors of KDR (VEGFR-2) kinase: An overview of structure activity relationships. Curr Top Med Chem. 2002;2:973-1000.
Campochiaro PA, the C99-PKC412-003 Study Group. Reduction of diabetic macular edema by oral administration of the kinase inhibitor PKC412. IOVS. 2004;45:922-31.
Carmeliet P, Rerreira V, Breier G, et al. Abnormal blood vessel development and lethality in embryos lacking a single VEGF allele. Nature. 1996;380:435-9.
Chen Y-S, Hackett SF, Schoenfeld C-L, Vinores MA, Vinores SA, Campochiaro PA. Localisation of vascular endothelial growth factor and its receptors to cells of vascular and avascular epiretinal membranes. Br J Ophthalmol. 1997;81:919-26.
Cousins SW, Espinosa-Heidmann DG, Csaky KG. Monocyte activation in patients with age-related macular degeneration - A biomarker of risk for choroidal neovascularization? Arch Ophthalmol. 2004;122(7):1013-8.
Csaky KG, Baffi JZ, Byrnes GA, et al. Recruitment of marrow-derived endothelial cells to experimental choroidal neovascularization by local expression of vascular endothelial growth factor. Exp Eye Res. 2004;78:1107-16.
Curtin ML, Frey RR, Heyman R, et al. Isoindolinone ureas: a novel class of KDR kinase inhibitors. Bioorg Med Chem Lett. 2004;14:4505-9.
De Vries C, Escobedo JA, Ueno H, Houck K, Ferrar N, Williams LT. The fins-like tyrosine kinase, a receptor for vascular endothelial growth factor. Science. 1992;255:989-91.
Dehmel U, Zaborski M, Meierhoff G, et al. Effects of FLT3 ligand on human leukemia cells. I. Proliferative response of myeloid leukemia cells. Leukemia. 1996;10:261-70.
Eriksson U, Alitalo K. VEGF receptor-1 stimulates stem-cell recruitment and new hope for angiogenesis therapies. Nat Med. 2002;8:775-7.
Espinosa-Heidmann DG, Caicedo A, Hernandez EP, Csaky KG, Cousins SW. Bone marrow-derived progenitor cells contribute to experimental choroidal neovascularization. IOVS. 2003;44(11):4914-19.
Eyetech Study Group. Preclinical and phase 1A clinical evaluation of an anti-VEGF pegylated aptamer (EYE001) for the treatment of exudative age-related macular degeneration. Retina. 2002;22:143-52.
Ferrara N, Davis-Smyth T. The biology of vascular endothelial growth factor. Endo Rev. 1997;18:4-25
Ferrara N, Gerber HP, LeCouter J. The biology of VEGF and its receptors. Nat Med. 2003;9:669-76.
Fong GH, Rossant J, Gertsenstein M, Breitman ML. Role of the Flt-1 receptor tyrosine kinase in regulating the assembly of vascular endothelium. Nature. 1995;376:66-70.
Fukumura D, Xavier R, Sugiura T, et al. Tumor induction of VEGF promoter activity in stromal cells. Cell. 1998;94:715-25.
George D, Platelet-derived growth factor receptors: A therapeutic target in solid tumors. Semin Oncol. 2001;28:27-33.
Grant MB, May WS, Caballero S, et al. Adult hematopoietic stem cells provide functional hemangioblast activity during retinal neovascularization. Nature Med. 2002; 6(8):607-12.
Griffioen AW, Molema G. Angiogenesis: Potentials for pharmacologic intervention in the treatment of cancer, cardovascular diseases, and chronic inflammation. Pharm Rev. 2000;52:237-68.
Gschwind A, Fischer OM, Ullrich A. The discovery of receptor tyrosine kinases: targets for cancer therapy. Nat Rev. 2004;4:361-70.
Hackett SF, Ozaki H, Strauss RW, et al. Angiopoietin 2 expression in the retina: Upregulation during physiologic and pathologic neovascularization. J Cell Physiol. 2000;184:275-84.
Hammes H-P, Lin J, Wagner, et al. Angiopoietin-2 causes pericyte dropout in the normal retina: Evidence for involvement in diabetic retinopathy. Diabetes. 2004;53:1104-10.
Hanahan D. Signaling vascular morphogenesis and maintenance. Science. 1997;277:48-50.
Hartnett ME, Lappas A, Darland D, McColm JR, Lovejoy S, D'Amore PA. Retinal pigment epithelium and endothelial cell interaction causes retinal pigment epithelial barrier disfunction via a soluble VEGF-dependent mechanism. Exp Eye Res. 2003;77:593-9.
Heinrich MC, Blanke CD, Druker BJ, Corless CL. Inhibition of KIT tyrosine kinase activity: A novel molecular approach to the treatment of KIT-positive malignancies. J Clin Oncol. 2002;20:1692-1703.
Hellström M, Kalén M, Lindahl P, Abramsson A, Betsholtz C. Role of PDGF-B and PDGFR-beta in recruitment of vascular smooth muscle cells and pericytes during embryonic blood vessel formation in the mouse. Development. 1999;126:3047-55.
Hunter T. Signaling-100 and beyond. Cell. 2000;100:113-127.
Inoue M, Hager JH, Ferrara N, Gerber HP, Hanahan D. VEGF-A has a critical, nonredundant role in angiogenic switching and pancreatic beta cell carcinogenesis. Cancer Cell. 2002;1:193-202.
Ishida S, Usui T, Yamashiro K, Kaji Y, Ahmed E, Carrasquillo KG, Amano S, Hida T, Oguchi Y, Adamis AP. VEGF164 is proinflammatory in the diabetic retina. IOYS. 2003;44(5):2155-62.
Ishida S, Usui T, Yamashiro K, et al. VEGF164 -mediated inflammation is required for pathological, but not physiological, ischemia-induced retinal neovascularization. J Exp Med. 2003;198(3):483-9.
Keck PJ, Hauser SD, Krivi G, Sanzo K, Warren T, Feder J, Connolly. Vascular permeability factor, an endothelial cell mitogen related to PDGF. Science. 1989;246:1309-12.
Kiyoi H, Naoe T. FLT3 in human hematologic malignancies. Leukemia Lymphoma. 2002;43:1541-7.
Kottaridis PD, Gale RE, Linch DC. Flt3 mutations and leukaemia. Br J Haem. 2003;122:523-38.
Krishnan J, Kirkin V, Steffen A, et al. Differential in vivo and in vitro expression of vascular endothelial growth factor (VEGF)-C and VEGF-D in tumors and its relationship to lymphatic metastasis in immunocompetent rats. Cancer Res. 2003; 63:713-22.
Krzystolik MG, Afshari MA, Adamis AP, et al. Prevention of experimental choroidal neovascularization with intravitreal anti-vascular endothelial growth factor antibody fragment. Arch Ophthalmol. 2002;120:338-46.
Kvanta A, Algvere PV, Berglin L, Seregard S. Subfoveal fibrovascular membranes in age-related macular degeneration express vascular endothelial growth factor. IOVS. 1996;37(9):1929-34.
Kwak N, Okamoto N, Wood JM, Campochiaro PA. VEGF is major stimulator in model of choroidal neovascularization. IOVS. 200;41:3158-64.
Lawrence DS, Niu J. Protein kinase inhibitors: The tyrosine-specific protein kinases. Pharmacol Ther. 1998;77(2):81-114.
Leung DW, Cachianes G, Kuang W-J, Goeddel DV, Ferrara N. Vascular endothelial growth factor is a secreted angiogenic mitogen. Science. 1989;246:1306-9.
Levis M, Small D. FLT3: It does matter in leukemia. Leukemia. 2003;17:1738-52.
Lindahl P, Johansson BR, Leveen P, Betsholtz C. Pericyte loss and microaneurysm formation in PDGF-B-deficient mice. Science. 1997;277:242-5.
Lutty GA, McLeod DS, Merges C, Diggs A, Plouet J. Localization of vascular endothelial growth factor in human retina and choroid. Arch Ophthalmol. 1996;114:971-7.
Lyden D, Hattor K, Dias S, et al. Impaired recruitment of bone-marrow-derived endothelial and hematopoietic precursor cells blocks tumor angiogenesis and growth. Nat Med. 2001;7:1194-1201.
Manley PW, Furet P, Bold G. Anthranilic acid amides: A novel class of antiangiogenic VEGF receptor kinase inhibitors. J Med Chem. 2002;45:5687-93.
Manning G, Whyte DB, Martinez R, Hunter T, Sudarsanam S. The protein kinase complement of the human genome. Science1 2002;298:1912-34.
McMahon G. Presentation given at the 1st International Symposium on Signal Transduction Modifiers in Cancer Therapy; September 23, 2002. Amsterdam, NL.
Millauer B, Wizigmann-Voos S, Schnurch H, Martinez R, Moller NP, Risau W, Ullrich A. High affinity VEGF binding and developmental expression suggest Flk-1 as a major regulator of vasculogenesis and angiogenesis. Cell. 1993;72:835-46.
Miller JW, Adamis AP, Shima DT, et al. Vascular endothelial growth factor / vascular permeability factor is temporally and spatially correlated with ocular angiogenesis in a primate model. Am J Pathol. 1994;145(3)574-84.
Mudhar HS, Pollock RA, Wang C, Stiles CD, Richardson WD. PDGF and its receptors in the developing rodent retina and optic nerve. Development. 1993;118:539-52.
Murukata C, Kaneko M, Gessner G, et al. Mixed lineage kinase activity of indolocarbazole analogues. Bioorg Med Chem Let. 2002;12:147-50.
Nakao M, Yokota S, Iwai T, et al. Internal tandem duplication of the flt3 gene found in acute myeloid leukemia. Leukemia. 1996;10:1911-8.
Natali PG, Nicotra MR, Sures I, Santoro E, Bigotti A, Ullrich A. Expression of c-kit receptro in normal and transformed human nonlymphoid tisues. Cancer Res. 1992;52:6139-43.
Oh H, Takagi H, Suzuma K, Otani A, Matsumura M, Honda Y. Hypoxia and vascular endothelial growth factor selectively up-regulate angiopoietin-2 in bovine microvascular endothelial cells. JBio Chem. 1999;274(22):15732-9.
Ohashi H, Takagi H, Koyama S, et al. Alterations in expression of angiopoietins and the Tie-2 receptor in the retina of streptozotocin induced diabetic rats. Mol Vis. 2004;10:608-17.
Ostman A, Heldin CH. Involvement of platelet-derived growth factor in disease: Development of specific antagonists. Adv Cancer Res. 2001;20:1-38.
Otani A, Takagi H, Oh H, Koyama S, Matsumura M, Honda Y. Expressions of angiopoietins and Tie2 in human choroidal neovascular membranes. IOVS. 1999;40(9)1912-20.
Ozaki H, Seo M-S, Ozaki K, et al. Blockade of vascular endothelial cell growth factor receptor signaling is sufficient to completely prevent retinal neovascularization. Am J Pathol. 200;156(2)697-707.
Pietras K, Rubin K, Sjoblom T, et al. Inhibition of PDGF receptor signaling in tumor stroma enhances antitumor effect of chemotherapy. Cancer Res. 2002;62:5476-84.
Ponten F, Ren Z, Nister M, Westermark B, Ponten J. Epithelial-stromal interactions in basal cell cancer: the PDGF system. J Inv Derm. 1994;102:304-9
Quinn TP, Peters KG, de Vries C, Ferrara N, Williams LT. Fetal liver kinase 1 is a receptor for vascular endothelial growth factor and is selectively expressed in vascular endothelium. Proc Natl Acad Sci. 1993;90:7533-7.
Rafii S, Lyden D, Benezra R, Hattori K, Heissig B. Vascular and haematopoietic stem cells: Novel targets for anti-angiogenesis therapy? Nat Rev Cancer. 2002;2:826-35.
Rak JW, St Croix BD, Kerbel RS. Consequences of angiogenesis for tumor progression, metastasis and cancer therapy. Anti-Cancer Drugs. 1995;6:3-18.
Reinmuth N, Liu W, Jung YD, et al. Induction of VEGF in perivascular cells defines a potential paracrine mechanism for endothelial cell survival. FASEB J. 2001;15:1239-41.
Robinson DR, Wu YM, Lin SF. The protein tyrosine kinase family of the human genome. Oncogene. 2000;19:5548-57.
Rosnet O, Buhring HJ, delapeyriere O, et al. Expression and signal transduction of the FLT3 tyrosine kinase receptor. Acta Haem. 1996;95:218-23.
Rosnet O, Schiff C, Pebusque MJ, et al. Human FLT3/FLK2 gene: cDNA cloning and expression in hematopoietic cells. Blood. 1993;82:1110-9.
Saishin Y, Saishin Y, Takahashi K, Silva RLE, Hylton D, Rudge JS, Wiegand SJ, Campochiaro PA. VEGF-TRAPR1R2 suppresses choroidal neovascularization and VEGF-induced breakdown of the blood-retinal barrier. J Cell Physiol. 2003;195:241-8.
Sarlos S, Rizkalla B, Moravski CJ, Cao Z, Cooper ME, Wilkinson-Berka JL. Retinal angiogenesis is mediated by an interaction between the angiotensin type 2 receptor, VEGF, and angiopoietin. Am J Pathol. 2003;163(3):879-87.
Sawyers CL. Finding the next Gleevec: FLT3 targeted kinase inhibitor therapy for acute myeloid leukemia. Canc Cell. 2002;1:413-5.
Schlessinger J. Cell signaling by receptor tyrosine kinases. Cell. 2000;103:211-25.
Seo MS, Kwak N, Ozaki H, et al. Dramatic inhibition of retinal and choroidal neovascularization by oral administration of a kinase inhibitor. Am J Pathol. 199;154(6):1743-53.
Shaheen RM, Tseng WW, Davis DW, et al. Tyrosine kinase inhibition of multiple angiogenic growth factor receptors improves survival in mice bearing colon cancer liver metastases by inhibition of endothelial cell survival mechanisms. Canc Res. 2001;61:1464-8.
Shalaby F, Rossant J, Yamaguchi TP, Gertsenstein M, Wu XF, Breitman ML, Schuh. Failure of blood-island formation and vasculogenesis in Flk-1-defecient mice.Nature. 1995;376:62-66.
Shen WY, Yu MJT, Barry CJ, Constable IJ, Rakoczy PE. Expression of cell adhesion molecules and vascular endothelial growth factor in experimental choroidal neovascularisation in the rat. Br J Opthalmol. 1998;82:1063-71.
Sherr CJ, Rettenmier CW, Sacca R, Roussel MF, Look AT, Stanley ER. The c-fms proto-oncogene product is related to the receptor for the mononuclear phagocyte growth factor, CSF-1. Cell. 1985;41:665-76.
Shima DT, Adamis AP, Ferrara N, Yeo K-T, Yeo T-K, Allende R, Folkman J, D'Amore PA. Hypoxic induction of endothelial cell growth factors in retinal cells: Identification and characterization of vascular endothelial growth factor (VEGF) as the mitogen. Mol Med. 1995;1(2):182-93.
Skobe M, Fusenig NE. Tumorigenic conversion of immortal human keratinocytes through stromal cell activation. Proc Natl Acad Sci. 1998;95:1050-5.
Sorbera LA, Leeson PA, Bayés M. Ranibizumab. Drugs Future. 2003;28(6):541-5.
Stirewalt DL, Radich JP. The role of FLT3 in haematopoietic malignancies. Nat Rev Cancer. 2003;3:650-65.
Stone J, Itin A, Alon T, Pe'er J, Gnessin H, Chan-Ling T, Keshet E. Development of retinal vasculature is mediated by hypoxia-induced vascular endothelial growth factor (VEGF) expression by neuroglia. J Neurosci. 1995;15(7):4738-47.
Takagi H, Koyama S, Seike H, et al. Potential role of the angiopoietin/Tie2 system in ischemia-induced retinal neovascularization. IOVS. 2003;44(1):393-4.02.
Terman BI, Dougher-Vermazen M, Carrion ME, Dimitrov D, Armellino DC, Gospodarowicz D, Bohlen P. Identificatio of the KDR tyrosine kinase as a receptor for vascular endothelial cell growth factor. Biochem Biophys Res Comm. 1992;187:1579-86.
Tian Q, Frierson HF Jr, Krystal GW, Moskaluk CA. Activating c-kit gene mutations in human germ cell tumors. Am J Pathol. 1999;154:1643-7.
Tolentino MJ, Miller JW, Gragoudas ES, et al. Vascular endothelial growth factor is sufficient to produce iris neovascularization and neovascular glaucoma in a nonhuman primate. Arch Ophthalmol. 1996;114:964-70.
Tolentino MJ, Miller JW, Gragoudas ES, et al. Intravitreous injections of vascular endothelial growth factor produce retinal ischemia and microangiopathy in an adult primate. Ophthalmol. 1996;103:1820-8.
Traxler P, Bold G, Buchdunger E, Caravatti G, et al. Tyrosine kinase inhibitors: From rational design to clinical trials. Med Res Rev. 2001;21(6):499-512.
Turner AM, Zsebo KM, Martin F, Jacobsen FW, Bennett LC, Broudy VC. Nonhematopoietic tumor cell lines express stem cell factor and display c-kit receptors. Blood. 1992;80:374-81.
Unsoeld AS, Junker B, Mazitschek R, et al. Local injeciton of receptor tyrosine kinase inhibitor MAE 87 reduces retinal neovascularization in mice. Mol Vis. 2004;10:468-75.
Waltenberger J, Claesson-Welsh L, Siegbahn A, Shibuya M, Heldin CH. Different signal transduction properties of KDR and Flt1, two receptors for vascular endothelial growth factor. J Bio Chem. 1994;269:26988-95.
Wang D, Huang HJ, Kazlauslcas A, Cavenee WK. Induction of vascular endothelial growth factor expression in endothelial cells by platelet-derived growth factor through the activation of phosphatidylinositol 3-kinase. Caracer Res. 1999;59:1464-72.
Werdich XQ, McCollum GW, Rajaratnam VS, Penn JS. Variable oxygen and retinal VEGF levels: correlation with incidence and severity of pathology in a rat model of oxygen-induced retinopathy. Exp Eye Res. 2004;79:623-30.
Wiesmann C, Fuh G, Christinger HW, EigenbrotC, Wells JA, de Vos, AM. Crystal structure at 1.7 Å resolution of VEGF in complex with domain-2 of the Flt-1 receptor. Cell. 1997;91:695-704.
Wilkinson-Berka JL, Babic S, De-Gooyer T, et al. Inhibition of platelet-derived growth factor promotes pericyte loss and angiogenesis in ischemic retinopathy. Am J Pathol. 2004;164(4):1263-73.
Witmer AN, Blaauwgeers HG, Weich HA, Alitalo K, Vrensen GFJM, Schlingemann RO. Altered expression patterns of VEGF receptors in human diabetic retina and in experimental VEGF-induced retinopathy in monkey. IOVS. 2002;43(3):849-57.
Yancopoulos GD, Davis S, Gale NW, Rudge JS, Wiegand SJ, Holash J. Vascular-specific growth factors and blood vessel formation. Nature. 2000;407:242-8.

### Clauses

1. A method for inhibiting ocular neovascularization and retinal edema, said method comprising administering to a patient in need thereof a composition comprising a therapeutically effective amount of a receptor tyrosine kinase inhibitor that blocks tyrosine autophosphorylation of VEGF receptor 1, VEGF receptor 2, VEGF receptor 3, Tie-2, PDGFR, c-KIT, Flt-3, and CSF-1R.
2. The method of claim 1, wherein the receptor tyrosine kinase inhibitor has an IC50 of from 0.1 nM to 250 nM for each of the receptors listed in claim 1.
3. The method of claim 1, wherein the receptor tyrosine kinase inhibitor blocks tyrosine autophosphorylation of Tie-2, PDGFR, and VEGF receptor 2 with an IC50 of from 0.1 nM to 200 nM for each receptor.
4. The method of claim 2, wherein the receptor tyrosine kinase inhibitor has an IC50 of from 0.1 nM to 100 nM for at least six of the receptor listed in claim 1.
5. The method of claim 4, wherein the receptor tyrosine kinase inhibitor has an IC50 of from 0.1 nM to 10 nM for at least four of the receptors listed in claim 1.
6. The method of claim 1, wherein the receptor tyrosine kinase inhibitor blocks tyrosine autophosphorylation of VEGF receptor 2, VEGF receptor 1, PDGFR, and Tie-2.
7. The method of claim 6, wherein the tyrosine kinase inhibitor has an IC50 of from 0.1 nM to 200 nM for each of the receptors listed in claim 6.
8. The method of claim 1, wherein the receptor tyrosine kinase inhibitor blocks tyrosine autophosphorylation of VEGF receptor 2, VEGF receptor 1, and Tie-2.
9. The method of claim 8, wherein the receptor tyrosine kinase inhibitor has an IC50 of from 0.1 nM to 200 nM for each of the receptors listed in claim 8.
10. The method of claim 1, wherein the receptor tyrosine kinase inhibitor blocks tyrosine autophosphorylation of VEGF receptor 2, VEGF receptor 1, and PDGFR.
11. The method of claim 10, wherein the receptor tyrosine kinase inhibitor has an IC50 of from 0.1 nM to 100 nM for each of the receptors listed in claim 10.
12. The method of claim 1, wherein the receptor tyrosine kinase inhibitor blocks tyrosine autophosphorylation of VEGF receptor 2 and Tie-2.
13. The method of claim 12, wherein the receptor tyrosine kinase inhibitor has an IC50 of from 0.1 nM to 200 nM for each of the receptors listed in claim 12.
14. The method of claim 13, wherein the receptor tyrosine kinase inhibitor has an IC50 of less than 10 nM for at least one of the receptors listed in claim 12.
15. The method of claim 1, wherein the receptor tyrosine kinase inhibitor blocks tyrosine autophosphorylation of VEGF receptor 2 and PDGFR.
16. The method of claim 15, wherein the receptor tyrosine kinase inhibitor has an IC50 of from 0.1 nM to 100 nM for each of the receptors listed in claim 15.
17. The method of claim 16, wherein the receptor tyrosine kinase inhibitor has an IC50 of less than 10 nM for at least one of the receptors listed in claim 15.
18. The method of claim 1, wherein the receptor tyrosine kinase inhibitor blocks tyrosine autophosphorylation of VEGF receptor 2, Tie-2, and PDGFR.
19. The method of claim 18, wherein the receptor tyrosine kinase inhibitor has an IC50 of between 0.1 nM and 200 nM for each of the receptors listed in claim 18.
20. The method of claim 19, wherein the receptor tyrosine kinase inhibitor has an IC50 of less than 10 nM for at least one of the receptors listed in claim 18.
21. The method of claim 1, wherein the receptor tyrosine kinase inhibitor is selected from the group consisting of
   N-[4-[3-amino-1H-indazol-4-yl)phenyl]-N'-(2-fluoro-5-methylphenyl)urea
   N-[4-(3-amino-1,2-benzisoxazol-4-yl)phenyl]-N'-(3-methylphenyl)urea;
   N-[4-(3-amino-1,2-benzisoxazol-4-yl)phenyl]-N'-[2-(trifluoromethyl)phenyl]urea;
   N-[4-(3-amino-1,2-benzisoxazol-4-yl)phenyl]-N'-(2-fluoro-5-methylphenyl)urea;
   N-[4-(3-amino-1,2-benzisoxazol-4-yl)phenyl]-N'-[3-(trifluoromethyl)phenyl]urea;
   N-[4-(3-amino-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-(3-methylphenyl)urea;
   N-[4-(3-amino-7-methoxy-1,2-benzisoxazol-4-yl)phenyl]-N'-[3-(trifluoromethyl)phenyl]urea;
   N-[4-(3-amino-7-methoxy-1,2-benzisoxazol-4-yl)phenyl]-N'-(3-chlorophenyl)urea;
   N-[4-(3-amino-7-methoxy-1,2-benzisoxazol-4-yl)phenyl]-N'-(2-fluoro-5-methylphenyl)urea;
   N-{4-[3-amino-7-(4-morpholinylmethyl)-1,2-benzisoxazol-4-yl]phenyl}-N'-[2-fluoro-5-(trifluoromethyl)phenyl]urea;
   N-{4-[3-amino-7-(4-morpholinylmethyl)-1,2-benzisoxazol-4-yl]phenyl}-N'-[3-(trifluoromethyl)phenyl]urea;
   N-{4-[3-amino-7-(4-morpholinylmethyl)-1,2-benzisoxazol-4-yl]phenyl}-N'-(3-chlorophenyl)urea;
   N-{4-[3-amino-7-(4-morpholinylmethyl)-1,2-benzisoxazol-4-yl]phenyl}-N'-(3-methylphenyl)urea;
   N-{4-[3-amino-7-(4-morpholinylmethyl)-1,2-benzisoxazol-4-yl]phenyl}-N'-(2-fluoro-5-methylphenyl)urea;
   N-{4-[3-amino-7-(4-morpholinylmethyl)-1,2-benzisoxazol-4-yl]phenyl}-N'-(3,5-dimethylphenyl)urea;
   N-{4-[3-amino-7-(4-morpholinylmethyl)-1,2-benzisoxazol-4-yl]phenyl}-N'-(3-phenoxyphenyl)urea;
   N-{4-[3-amino-7-(4-morpholinylmethyl)-1,2-benzisoxazol-4-yl]phenyl}-N'-(3-bromophenyl)urea;
   N-(4-{3-amino-7-[2-(4-morpholinyl)ethoxy]-1,2-benzisoxazol-4-yl}phenyl)-N'-[3-(trifluoromethyl)phenyl]urea;
   N-(4-{3-amino-7-[2-(4-morpholinyl)ethoxy]-1,2-benzisoxazol-4-yl}phenyl)-N'-(2-fluoro-5-methylphenyl)urea;
   N-(4-{3-amino-7-[2-(4-morpholinyl)ethoxy]-1,2-benzisoxazol-4-yl}phenyl)-N'-[2-fluoro-5-(trifluoromethyl)phenyl]urea;
   N-(4-{3-amino-7-[2-(4-morpholinyl)ethoxy]-1,2-benzisoxazol-4-yl}phenyl)-N'-(3-methylphenyl)urea;
   N-[4-(3-amino-1,2-benzisoxazol-4-yl)phenyl]-N'-(3,5-dimethylphenyl)urea;
   N-[4-(3-amino-1,2-benzisoxazol-4-yl)phenyl]-N'-phenylurea;
   N-[4-(3-amino-1,2-benzisoxazol-4-yl)phenyl]-N'-(4-methylphenyl)urea;
   N-[4-(3-amino-1,2-benzisoxazol-4-yl)phenyl]-N'-(3-cyanophenyl)urea;
   N-[4-(3-amino-1,2-benzisoxazol-4-yl)phenyl]-N'-[4-fluoro-3-(trifluoromethyl)phenyl]urea;
   N-[4-(3-amino-1,2-benzisoxazol-4-yl)phenyl]-N'-(3-bromophenyl)urea;
   N-[4-(3-amino-1,2-benzisoxazol-4-yl)phenyl]-N'-(3-chlorophenyl)urea;
   N-[4-(3-amino-1,2-benzisoxazol-4-yl)phenyl]-N'-(3-ethylphenyl)urea;
   N-[4-(3-amino-1,2-benzisoxazol-4-yl)phenyl]-N'-[4-(trifluoromethyl)phenyl]urea;
   N-[4-(3-amino-1,2-benzisoxazol-4-yl)phenyl]-N'-(3-fluoro-4-methylphenyl)urea;
   N-[4-(3-amino-1,2-benzisoxazol-4-yl)phenyl]-N'-(3-fluorophenyl)urea;
   N-[4-(3-amino-1,2-benzisoxazol-4-yl)phenyl]-N'-(3,5-difluorophenyl)urea;
   N-[4-(3-amino-1,2-benzisoxazol-4-yl)phenyl]-N'-(3-methoxyphenyl)urea;
   N-[4-(3-amino-1,2-benzisoxazol-4-yl)phenyl]-N'-(4-methoxyphenyl)urea;
   N-[4-(3-amino-1,2-benzisoxazol-4-yl)phenyl]urea;
   N-[4-(3-amino-1,2-benzisoxazol-4-yl)phenyl]-N'-(3-nitrophenyl)urea;
   N-[4-(3-amino-1,2-benzisoxazol-4-yl)phenyl]-N'-(4-fluorophenyl)urea;
   N-[4-(3-amino-1,2-benzisoxazol-4-yl)phenyl]-N'-(2-fluorophenyl)urea;
   N-[4-(3-amino-1,2-benzisoxazol-4-yl)phenyl]-N'-(3-chloro-4-fluorophenyl)urea;
   N-[4-(3-amino-1,2-benzisoxazol-4-yl)phenyl]-N'-(3-chloro-4-methoxyphenyl)urea;
   N-[4-(3-amino-1,2-benzisoxazol-4-yl)phenyl]-N'-[4-(dimethylamino)phenyl]urea;
   N-[4-(3-amino-1,2-benzisoxazol-4-yl)phenyl]-N'-[4-(trifluoromethoxy)phenyl]urea;
   N-[4-(3-amino-1,2-benzisoxazol-4-yl)phenyl]-N'-[2-(trifluoromethoxy)phenyl]urea;
   N-[4-(3-amino-1,2-benzisoxazol-4-yl)phenyl]-N'-[3,5-bis(trifluoromethyl)phenyl]urea;
   N-[4-(3-amino-1,2-benzisoxazol-4-yl)phenyl]-N'-(3-chloro-4-methylphenyl)urea;
   N-[4-(3-amino-7-methoxy-1,2-benzisoxazol-4-yl)phenyl]-N'-[3,5-bis(trifluoromethyl)phenyl]urea;
   N-[4-(3-amino-7-methoxy-1,2-benzisoxazol-4-yl)phenyl]-N'-[4-(trifluoromethoxy)phenyl]urea;
   N-[4-(3-amino-7-methoxy-1,2-benzisoxazol-4-yl)phenyl]-N'-(3-fluorophenyl)urea;
   N-[4-(3-amino-7-methoxy-1,2-benzisoxazol-4-yl)phenyl]-N'-(3-methoxyphenyl)urea;
   N-[4-(3-amino-7-methoxy-1,2-benzisoxazol-4-yl)phenyl]-N'-(3,5-difluorophenyl)urea;
   N-[4-(3-amino-7-methoxy-1,2-benzisoxazol-4-yl)phenyl]-N'-(4-methylphenyl)urea;
   N-[4-(3-amino-7-methoxy-1,2-benzisoxazol-4-yl)phenyl]-N'-(3-bromophenyl)urea;
   N-[4-(3-amino-7-methoxy-1,2-benzisoxazol-4-yl)phenyl]-N'-(3,5-dimethylphenyl)urea;
   N-[4-(3-amino-7-methoxy-1,2-benzisoxazol-4-yl)phenyl]-N'-[4-(dimethylamino)phenyl]urea;
   N-[4-(3-amino-7-methyl-1,2-benzisoxazol-4-yl)phenyl]-N'-(3-methylphenyl)urea;
   N-[4-(3-amino-7-methyl-1,2-benzisoxazol-4-yl)phenyl]-N'-(3-chlorophenyl)urea;
   N-[4-(3-amino-7-methyl-1,2-benzisoxazol-4-yl)phenyl]-N'-(2-fluoro-5-methylphenyl)urea;
   N-[4-(3-amino-7-methyl-1,2-benzisoxazol-4-yl)phenyl]-N'-[2-fluoro-5-(trifluoromethyl)phenyl]urea;
   N-[4-(3-amino-7-methyl-1,2-benzisoxazol-4-yl)phenyl]-N'-[3-(trifluoromethyl)phenyl]urea;
   N-[4-(3-amino-7-methyl-1,2-benzisoxazol-4-yl)phenyl]-N'-(3,5-dimethylphenyl)urea;
   N-[4-(3-amino-7-methyl-1,2-benzisoxazol-4-yl)phenyl]-N'-(3-ethylphenyl)urea;
   N-[4-(3-amino-7-methyl-1,2-benzisoxazol-4-yl)phenyl]-N'-(4-methylphenyl)urea;
   N-[4-(3-amino-7-methyl-1,2-benzisoxazol-4-yl)phenyl]-N'-[4-(trifluoromethoxy)phenyl]urea;
   N-[4-(3-amino-7-methyl-1,2-benzisoxazol-4-yl)phenyl]-N'-(3-fluoro-4-methylphenyl)urea;
   N-[4-(3-ammo-7-methyl-1,2-benzisoxazol-4-yl)phenyl]-N'-(3-methoxyphenyl)urea;
   N-[4-(3-amino-7-methyl-1,2-benzisoxazol-4-yl)phenyl]-N'-phenylurea;
   N-[4-(3-amino-7-methyl-1,2-benzisoxazol-4-yl)phenyl]-N'-[3,5-bis(trifluoromethyl)phenyl]urea;
   N-[4-(3-amino-7-methyl-1,2-benzisoxazol-4-yl)phenyl]-N'-(3-bromophenyl)urea;
   N-[4-(3-amino-7-methyl-1,2-benzisoxazol-4-yl)phenyl]-N'-(3-fluorophenyl)urea;
   N-[4-(3-amino-7-methoxy-1,2-benzisoxazol-4-yl)phenyl]-N'-[4-fluoro-3-(trifluoromethyl)phenyl]urea;
   N-[4-(3-amino-7-methoxy-1,2-benzisoxazol-4-yl)phenyl]-N'-(4-fluoro-3-methylphenyl)urea;
   N-[4-(3-amino-7-fluoro-1,2-benzisoxazol-4-yl)phenyl]-N'-[3-(trifluoromethyl)phenyl]urea;
   N-[4-(3-amino-7-fluoro-1,2-benzisoxazol-4-yl)phenyl]-N'-(3-chlorophenyl)urea;
   N-[4-(3-amino-7-fluoro-1,2-benzisoxazol-4-yl)phenyl]-N'-[4-fluoro-3-(trifluoromethyl)phenyl]urea;
   N-[4-(3-amino-7-fluoro-1,2-benzisoxazol-4-yl)phenyl]-N'-(3-methylphenyl)urea;
   N-[4-(3-amino-7-fluoro-1,2-benzisoxazol-4-yl)phenyl]-N'-[2-fluoro-5-(trifluoromethyl)phenyl]urea;
   N-[4-(3-amino-7-fluoro-1,2-benzisoxazol-4-yl)phenyl]-N'-(2-fluoro-5-methylphenyl)urea;
   N-{4-[3-amino-7-(trifluoromethoxy)-1,2-benzisoxazol-4-yl]phenyl}-N'-[2-fluoro-5-(trifluoromethyl)phenyl]urea;
   N-{4-[3-amino-7-(trifluoromethoxy)-1,2-benzisoxazol-4-yl]phenyl}-N'-[3-(trifluoromethyl)phenyl]urea;
   N-{4-[3-amino-7-(trifluoromethoxy)-1,2-benzisoxazol-4-yl]phenyl}-N'-(2-fluoro-5-methylphenyl)urea;
   N-{4-[3-amino-7-(trifluoromethoxy)-1,2-benzisoxazol-4-yl]phenyl}-N'-(3-chlorophenyl)urea;
   N-{4-[3-amino-7-(trifluoromethoxy)-1,2-benzisoxazol-4-yl]phenyl}-N'-(3-bromophenyl)urea;
   N-{4-[3-amino-7-(trifluoromethoxy)-1,2-benzisoxazol-4-yl]phenyl}-N'-[4-fluoro-3-(trifluoromethyl)phenyl]urea; and
   N-{4-[3-amino-7-(trifluoromethoxy)-1,2-benzisoxazol-4-yl]phenyl}-N'-(4-fluoro-3-methylphenyl)urea.
22. The method of claim 21, wherein said compound is N-[4-[3-amino-1H-indazol-4-yl]phenyl]-N'-(2-fluoro-5-methylphenyl)urea.
23. The method of claim 1, wherein said composition is administered via a method selected from the group consisting of topical, subconjunctival, periocular, retrobulbar, subtenon, intracameral, intravitreal, intraocular, subretinal, posterior juxtascleral, and suprachoroidal administration.
24. The method of claim 23, wherein the composition is administered via intravitreal or subtenon injection of a solution or suspension.
25. The method of claim 23, wherein the composition is administered via intravitreal or subtenon placement of a device.
26. The method of claim 23, wherein the composition is administered via topical ocular administration of a solution or suspension.
27. The method of claim 23, wherein the composition is administered via posterior juxtascleral administration of a gel.
28. The method of claim 23, wherein the composition is administered via intravitreal administration of a bioerodible implant.
29. A method for causing regression of neovascularization, said method comprising administering to a patient in need thereof a composition comprising a therapeutically effective amount of a receptor tyrosine kinase inhibitor that blocks tyrosine autophosphorylation of VEGF receptor 1, VEGF receptor 2, VEGF receptor 3, Tie-2, PDGFR, c-KIT, Flt-3, and CSF-1R.
30. The method of claim 29, wherein the receptor tyrosine kinase inhibitor has an IC50 of from 0.1 nM to 250 nM for each of the receptors listed in claim 29.
31. The method of claim 29, wherein the receptor tyrosine kinase inhibitor blocks tyrosine autophosphorylation of Tie-2, PDGFR, and VEGF receptor 2 with an IC50 of from 0.1 nM to 200 nM for each receptor.
32. The method of claim 31, wherein the receptor tyrosine kinase inhibitor has an IC50 of from 0.1 nM to 100 nM for at least six of the receptors listed in claim 29.
33. The method of claim 32, wherein the receptor tyrosine kinase inhibitor has an IC50 of from 0.1 nM to 10 nM for at least four of the receptors listed in claim 29.
34. The method of claim 29, wherein the receptor tyrosine kinase inhibitor blocks tyrosine autophosphorylation of VEGF receptor 2, VEGF receptor 1, PDGFR, and Tie-2.
35. The method of claim 34, wherein the tyrosine kinase inhibitor has an IC50 of from 0.1 nM to 200 nM for each of the receptors listed in claim 34.
36. The method of claim 29, wherein the receptor tyrosine kinase inhibitor blocks tyrosine autophosphorylation of VEGF receptor 2, VEGF receptor 1, and Tie-2.
37. The method of claim 36, wherein the receptor tyrosine kinase inhibitor has an IC50 of from 0.1 nM to 200 nM for each of the receptors listed in claim 36.
38. The method of claim 29, wherein the receptor tyrosine kinase inhibitor blocks tyrosine autophosphorylation of VEGF receptor 2, VEGF receptor 1, and PDGFR.
39. The method of claim 38, wherein the receptor tyrosine kinase inhibitor has an IC50 of from 0.1 nM to 100 nM for each of the receptors listed in claim 38.
40. The method of claim 29, wherein the receptor tyrosine kinase inhibitor blocks tyrosine autophosphorylation of VEGF receptor 2 and Tie-2.
41. The method of claim 40, wherein the receptor tyrosine kinase inhibitor has an IC50 of from 0.1 nM to 200 nM for each of the receptors listed in claim 40.
42. The method of claim 41, wherein the receptor tyrosine kinase inhibitor has an IC50 of less than 10 nM for at least one of the receptors listed in claim 40.
43. The method of claim 29, wherein the receptor tyrosine kinase inhibitor blocks tyrosine autophosphorylation of VEGF receptor 2 and PDGFR.
44. The method of claim 43, wherein the receptor tyrosine kinase inhibitor has an IC50 of from 0.1 nM to 100 nM for each of the receptors listed in claim 43.
45. The method of claim 44, wherein the receptor tyrosine kinase inhibitor has an IC50 of less than 10 nM for at least one of the receptors listed in claim 43.
46. The method of claim 29, wherein the receptor tyrosine kinase inhibitor blocks tyrosine autophosphorylation of VEGF receptor 2, Tie-2, and PDGFR.
47. The method of claim 46, wherein the receptor tyrosine kinase inhibitor has an IC50 of between 0.1 nM and 200 nM for each of the receptors listed in claim 46.
48. The method of claim 47, wherein the receptor tyrosine kinase inhibitor has an IC50 of less than 10 nM for at least one of the receptors listed in claim 46.
49. The method of claim 29, wherein the receptor tyrosine kinase inhibitor is selected from the group consisting of
   N-[4-[3-amino-1H-indazol-4-yl)phenyl]-N'-(2-fluoro-5-methylphenyl)urea
   N-[4-(3-amino-1,2-benzisoxazol-4-yl)phenyl]-N'-(3-methylphenyl)urea;
   N-[4-(3-amino-1,2-benzisoxazol-4-yl)phenyl]-N'-[2-(trifluoromethyl)phenyl]urea;
   N-[4-(3-amino-1,2-benzisoxazol-4-yl)phenyl]-N'-(2-fluoro-5-methylphenyl)urea;
   N-[4-(3-amino-1,2-benzisoxazol-4-yl)phenyl]-N'-[3-(trifluoromethyl)phenyl]urea;
   N-[4-(3-amino-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-(3-methylphenyl)urea;
   N-[4-(3-amino-7-methoxy-1,2-benzisoxazol-4-yl)phenyl]-N'-[3-(trifluoromethyl)phenyl]urea;
   N-[4-(3-amino-7-methoxy-1,2-benzisoxazol-4-yl)phenyl]-N'-(3-chlorophenyl)urea;
   N-[4-(3-amino-7-methoxy-1,2-benzisoxazol-4-yl)phenyl]-N'-(2-fluoro-5-methylphenyl)urea;
   N-{4-[3-amino-7-(4-morpholinylmethyl)-1,2-benzisoxazol-4-yl]phenyl}-N'-[2-fluoro-5-(trifluoromethyl)phenyl]urea;
   N-{4-[3-ammo-7-(4-morpholinylmethyl)-1,2-benzisoxazol-4-yl]phenyl}-N'-[3-(trifluoromethyl)phenyl]urea;
   N-{4-[3-amino-7-(4-morpholinylmethyl)-1,2-benzisoxazol-4-yl]phenyl}-N'-(3-chlorophenyl)urea;
   N-{4-[3-ammo-7-(4-morpholinylmethyl)-1,2-benzisoxazol-4-yl]phenyl}-N'-(3-methylphenyl)urea;
   N-{4-[3-amino-7-(4-morpholinylmethyl)-1,2-benzisoxazol-4-yl]phenyl}-N'-(2-fluoro-5-methylphenyl)urea;
   N-{4-[3-amino-7-(4-morpholinylmethyl)-1,2-benzisoxazol-4-yl]phenyl}-N'-(3,5-dimethylphenyl)urea;
   N-{4-[3-amino-7-(4-morpholinylmethyl)-1,2-benzisoxazol-4-yl]phenyl}-N'-(3-phenoxyphenyl)urea;
   N-{4-[3-amino-7-(4-morpholinylmethyl)-1,2-benzisoxazol-4-yl]phenyl}-N'-(3-bromophenyl)urea;
   N-(4-{3-amino-7-[2-(4-morpholmyl)ethoxy]-1,2-benzisoxazol-4-yl}phenyl)-N'-[3-(trifluoromethyl)phenyl]urea;
   N-(4-{3-amino-7-[2-(4-morpholinyl)ethoxy]-1,2-benzisoxazol-4-yl}phenyl)-N'-(2-fluoro-5-methylphenyl)urea;
   N-(4-{3-amino-7-[2-(4-morpholinyl)ethoxy]-1,2-benzisoxazol-4-yl}phenyl)-N'-[2-fluoro-5-(trifluoromethyl)phenyl]urea;
   N-(4-{3-amino-7-[2-(4-morpholinyl)ethoxy]-1,2-benzisoxazol-4-yl}phenyl)-N'-(3-methylphenyl)urea;
   N-[4-(3-amino-1,2-benzisoxazol-4-yl)phenyl]-N'-(3,5-dimethylphenyl)urea;
   N-[4-(3-amino-1,2-benzisoxazol-4-yl)phenyl]-N'-phenylurea;
   N-[4-(3-amino-1,2-benzisoxazol-4-yl)phenyl]-N'-(4-methylphenyl)urea;
   N-[4-(3-amino-1,2-benzisoxazol-4-yl)phenyl]-N'-(3-cyanophenyl)urea;
   N-[4-(3-amino-1,2-benzisoxazol-4-yl)phenyl]-N'-[4-fluoro-3-(trifluoromethyl)phenyl]urea;
   N-[4-(3-amino-1,2-benzisoxazol-4-yl)phenyl]-N'-(3-bromophenyl)urea;
   N-[4-(3-amino-1,2-benzisoxazol-4-yl)phenyl]-N'-(3-chlorophenyl)urea;
   N-[4-(3-amino-1,2-benzisoxazol-4-yl)phenyl]-N'-(3-ethylphenyl)urea;
   N-[4-(3-amino-1,2-benzisoxazol-4-yl)phenyl]-N'-[4-(trifluoromethyl)phenyl]urea;
   N-[4-(3-amino-1,2-benzisoxazol-4-yl)phenyl]-N'-(3-fluoro-4-methylphenyl)urea;
   N-[4-(3-amino-1,2-benzisoxazol-4-yl)phenyl]-N'-(3-fluorophenyl)urea;
   N-[4-(3-amino-1,2-benzisoxazol-4-yl)phenyl]-N'-(3,5-difluorophenyl)urea;
   N-[4-(3-amino-1,2-benzisoxazol-4-yl)phenyl]-N'-(3-methoxyphenyl)urea;
   N-[4-(3-amino-1,2-benzisoxazol-4-yl)phenyl]-N'-(4-methoxyphenyl)urea;
   N-[4-(3-amino-1,2-benzisoxazol-4-yl)phenyl]urea;
   N-[4-(3-amino-1,2-benzisoxazol-4-yl)phenyl]-N'-(3-nitrophenyl)urea;
   N-[4-(3-amino-1,2-benzisoxazol-4-yl)phenyl]-N'-(4-fluorophenyl)urea;
   N-[4-(3-amino-1,2-benzisoxazol-4-yl)phenyl]-N'-(2-fluorophenyl)urea;
   N-[4-(3-amino-1,2-benzisoxazol-4-yl)phenyl]-N'-(3-chloro-4-fluorophenyl)urea;
   N-[4-(3-amino-1,2-benzisoxazol-4-yl)phenyl]-N'-(3-chloro-4-methoxyphenyl)urea;
   N-[4-(3-amino-1,2-benzisoxazol-4-yl)phenyl]-N'-[4-(dimethylamino)phenyl]urea;
   N-[4-(3-amino-1,2-benzisoxazol-4-yl)phenyl]-N'-[4-(trifluoromethoxy)phenyl]urea;
   N-[4-(3-amino-1,2-benzisoxazol-4-yl)phenyl]-N'-[2-(trifluoromethoxy)phenyl]urea;
   N-[4-(3-amino-1,2-benzisoxazol-4-yl)phenyl]-N'-[3,5-bis(trifluoromethyl)phenyl]urea;
   N-[4-(3-amino-1,2-benzisoxazol-4-yl)phenyl]-N'-(3-chloro-4-methylphenyl)urea;
   N-[4-(3-amino-7-methoxy-1,2-benzisoxazol-4-yl)phenyl]-N'-[3,5-bis(trifluoromethyl)phenyl]urea;
   N-[4-(3-amino-7-methoxy-1,2-benzisoxazol-4-yl)phenyl]-N'-[4-(trifluoromethoxy)phenyl]urea;
   N-[4-(3-amino-7-methoxy-1,2-benzisoxazol-4-yl)phenyl]-N'-(3-fluorophenyl)urea;
   N-[4-(3-amino-7-methoxy-1,2-benzisoxazol-4-yl)phenyl]-N'-(3-methoxyphenyl)urea;
   N-[4-(3-amino-7-methoxy-1,2-benzisoxazol-4-yl)phenyl]-N'-(3,5-difluorophenyl)urea;
   N-[4-(3-amino-7-methoxy-1,2-benzisoxazol-4-yl)phenyl]-N'-(4-methylphenyl)urea;
   N-[4-(3-amino-7-methoxy-1,2-benzisoxazol-4-yl)phenyl]-N'-(3-bromophenyl)urea;
   N-[4-(3-amino-7-methoxy-1,2-benzisoxazol-4-yl)phenyl]-N'-(3,5-dimethylphenyl)urea;
   N-[4-(3-amino-7-methoxy-1,2-benzisoxazol-4-yl)phenyl]-N'-[4-(dimethylamino)phenyl]urea;
   N-[4-(3-amino-7-methyl-1,2-benzisoxazol-4-yl)phenyl]-N'-(3-methylphenyl)urea;
   N-[4-(3-amino-7-methyl-1,2-benzisoxazol-4-yl)phenyl]-N'-(3-chlorophenyl)urea;
   N-[4-(3-amino-7-methyl-1,2-benzisoxazol-4-yl)phenyl]-N'-(2-fluoro-5-methylphenyl)urea;
   N-[4-(3-amino-7-methyl-1,2-benzisoxazol-4-yl)phenyl]-N'-[2-fluoro-5-(trifluoromethyl)phenyl]urea;
   N-[4-(3-amino-7-methyl-1,2-benzisoxazol-4-yl)phenyl]-N'-[3-(trifluoromethyl)phenyl]urea;
   N-[4-(3-amino-7-methyl-1,2-benzisoxazol-4-yl)phenyl]-N'-(3,5-dimethylphenyl)urea;
   N-[4-(3-amino-7-methyl-1,2-benzisoxazol-4-yl)phenyl]-N'-(3-ethylphenyl)urea;
   N-[4-(3-amino-7-methyl-1,2-benzisoxazol-4-yl)phenyl]-N'-(4-methylphenyl)urea;
   N-[4-(3-amino-7-methyl-1,2-benzisoxazol-4-yl)phenyl]-N'-[4-(trifluoromethoxy)phenyl]urea;
   N-[4-(3-amino-7-methyl-1,2-benzisoxazol-4-yl)phenyl]-N'-(3-fluoro-4-methylphenyl)urea;
   N-[4-(3-amino-7-methyl-1,2-benzisoxazol-4-yl)phenyl]-N'-(3-methoxyphenyl)urea;
   N-[4-(3-amino-7-methyl-1,2-benzisoxazol-4-yl)phenyl]-N'-phenylurea;
   N-[4-(3-amino-7-methyl-1,2-benzisoxazol-4-yl)phenyl]-N'-[3,5-bis(trifluoromethyl)phenyl]urea;
   N-[4-(3-amino-7-methyl-1,2-benzisoxazol-4-yl)phenyl]-N'-(3-bromophenyl)urea;
   N-[4-(3-amino-7-methyl-1,2-benzisoxazol-4-yl)phenyl]-N'-(3-fluorophenyl)urea;
   N-[4-(3-amino-7-methoxy-1,2-benzisoxazol-4-yl)phenyl]-N'-[4-fluoro-3-(trifluoromethyl)phenyl]urea;
   N-[4-(3-amino-7-methoxy-1,2-benzisoxazol-4-yl)phenyl]-N'-(4-fluoro-3-methylphenyl)urea;
   N-[4-(3-amino-7-fluoro-1,2-benzisoxazol-4-yl)phenyl]-N'-[3-(trifluoromethyl)phenyl]urea;
   N-[4-(3-amino-7-fluoro-1,2-benzisoxazol-4-yl)phenyl]-N'-(3-chlorophenyl)urea;
   N-[4-(3-amino-7-fluoro-1,2-benzisoxazol-4-yl)phenyl]-N'-[4-fluoro-3-(trifluoromethyl)phenyl]urea;
   N-[4-(3-amino-7-fluoro-1,2-benzisoxazol-4-yl)phenyl]-N'-(3-methylphenyl)urea;
   N-[4-(3-amino-7-fluoro-1,2-benzisoxazol-4-yl)phenyl]-N'-[2-fluoro-5-(trifluoromethyl)phenyl]urea;
   N-[4-(3-amino-7-fluoro-1,2-benzisoxazol-4-yl)phenyl]-N'-(2-fluoro-5-methylphenyl)urea;
   N-{4-[3-amino-7-(trifluoromethoxy)-1,2-benzisoxazol-4-yl]phenyl}-N'-[2-fluoro-5-(trifluoromethyl)phenyl]urea;
   N-{4-[3-amino-7-(trifluoromethoxy)-1,2-benzisoxazol-4-yl]phenyl}-N'-[3-(trifluoromethyl)phenyl]urea;
   N-{4-[3-amino-7-(trifluoromethoxy)-1,2-benzisoxazol-4-yl]phenyl}-N'-(2-fluoro-5-methylphenyl)urea;
   N-{4-[3-amino-7-(trifluoromethoxy)-1,2-benzisoxazol-4-yl]phenyl}-N'-(3-chlorophenyl)urea;
   N-{4-[3-amino-7-(trifluoromethoxy)-1,2-benzisoxazol-4-yl]phenyl}-N'-(3-bromophenyl)urea;
   N-{4-[3-amino-7-(trifluoromethoxy)-1,2-benzisoxazol-4-yl]phenyl}-N'-[4-fluoro-3-(trifluoromethyl)phenyl]urea; and
   N-{4-[3-amino-7-(trifluoromethoxy)-1,2-benzisoxazol-4-yl]phenyl}-N'-(4-fluoro-3-methylphenyl)urea.
50. The method of claim 49, wherein said compound is N-[4-[3-amino-1H-indazol-4-yl]phenyl]-N'-(2-fluoro-5-methylphenyl)urea.
51. The method of claim 29, wherein said composition is administered via a method selected from the group consisting of topical, subconjunctival, periocular, retrobulbar, subtenon, intracameral, intravitreal, intraocular, subretinal, posterior juxtascleral, and suprachoroidal administration.
52. The method of claim 51, wherein the composition is administered via intravitreal or subtenon injection of a solution or suspension.
53. The method of claim 51, wherein the composition is administered via intravitreal or subtenon placement of a device.
54. The method of claim 51, wherein the composition is administered via topical ocular administration of a solution or suspension.
55. The method of claim 51, wherein the composition is administered via posterior juxtascleral administration of a gel.
56. The method of claim 51, wherein the composition is administered via intravitreal administration of a bioerodible implant.
57. A method for inhibiting retinal edema, said method comprising administering to a patient in need thereof a composition comprising a therapeutically effective amount of a receptor tyrosine kinase inhibitor that blocks tyrosine autophosphorylation of VEGF receptor 1, VEGF receptor 2, VEGF receptor 3, Tie-2, PDGFR, c-KIT, Flt-3, and CSF-1R.
58. The method of claim 57, wherein the receptor tyrosine kinase inhibitor has an IC50 of from 0.1 nM to 250 nM for each of the receptors listed in claim 57.
59. The method of claim 57, wherein the receptor tyrosine kinase inhibitor blocks tyrosine autophosphorylation of Tie-2, PDGFR, and VEGF receptor 2 with an IC50 of from 0.1 nM to 200 nM for each receptor.
60. The method of claim 59, wherein the receptor tyrosine kinase inhibitor has an IC50 of from 0.1 nM to 100 nM for at least six of the receptor listed in claim 57.
61. The method of claim 60, wherein the receptor tyrosine kinase inhibitor has an IC50 of from 0.1 nM to 10 nM for at least four of the receptors listed in claim 57.
62. The method of claim 57, wherein the receptor tyrosine kinase inhibitor blocks tyrosine autophosphorylation of VEGF receptor 2, VEGF receptor 1, PDGFR, and Tie-2.
63. The method of claim 62, wherein the tyrosine kinase inhibitor has an IC50 of from 0.1 nM to 200 nM for each of the receptors listed in claim 62.
64. The method of claim 57, wherein the receptor tyrosine kinase inhibitor blocks tyrosine autophosphorylation of VEGF receptor 2, VEGF receptor 1, and Tie-2.
65. The method of claim 64, wherein the receptor tyrosine kinase inhibitor has an IC50 of from 0.1 nM to 200 nM for each of the receptors listed in claim 64.
66. The method of claim 57, wherein the receptor tyrosine kinase inhibitor blocks tyrosine autophosphorylation of VEGF receptor 2, VEGF receptor 1, and PDGFR.
67. The method of claim 66, wherein the receptor tyrosine kinase inhibitor has an IC50 of from 0.1 nM to 100 nM for each of the receptors listed in claim 66.
68. The method of claim 57, wherein the receptor tyrosine kinase inhibitor blocks tyrosine autophosphorylation of VEGF receptor 2 and Tie-2.
69. The method of claim 68, wherein the receptor tyrosine kinase inhibitor has an IC50 of from 0.1 nM to 200 nM for each of the receptors listed in claim 68.
70. The method of claim 69, wherein the receptor tyrosine kinase inhibitor has an IC50 of less than 10 nM for at least one of the receptors listed in claim 68.
71. The method of claim 57, wherein the receptor tyrosine kinase inhibitor blocks tyrosine autophosphorylation of VEGF receptor 2 and PDGFR.
72. The method of claim 71, wherein the receptor tyrosine kinase inhibitor has an IC50 of from 0.1 nM to 100 nM for each of the receptors listed in claim 71.
73. The method of claim 72, wherein the receptor tyrosine kinase inhibitor has an IC50 of less than 10 nM for at least one of the receptors listed in claim 71.
74. The method of claim 57, wherein the receptor tyrosine kinase inhibitor blocks tyrosine autophosphorylation of VEGF receptor 2, Tie-2, and PDGFR.
75. The method of claim 74, wherein the receptor tyrosine kinase inhibitor has an IC50 of between 0.1 nM and 200 nM for each of the receptors listed in claim 74.
76. The method of claim 75, wherein the receptor tyrosine kinase inhibitor has an IC50 of less than 10 nM for at least one of the receptors listed in claim 74.
77. The method of claim 57, wherein the receptor tyrosine kinase inhibitor is selected from the group consisting of
   N-[4-[3-amino-1H-indazol-4-yl)phenyl]-N'-(2-fluoro-5-methylphenyl)urea
   N-[4-(3-amino-1,2-benzisoxazol-4-yl)phenyl]-N'-(3-methylphenyl)urea;
   N-[4-(3-amino-1,2-benzisoxazol-4-yl)phenyl]-N'-[2-(trifluoromethyl)phenyl]urea;
   N-[4-(3-amino-1,2-benzisoxazol-4-yl)phenyl]-N'-(2-fluoro-5-methylphenyl)urea;
   N-[4-(3-amino-1,2-benzisoxazol-4-yl)phenyl]-N'-[3-(trifluoromethyl)phenyl]urea;
   N-[4-(3-amino-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-(3-methylphenyl)urea;
   N-[4-(3-amino-7-methoxy-1,2-benzisoxazol-4-yl)phenyl]-N'-[3-(trifluoromethyl)phenyl]urea;
   N-[4-(3-amino-7-methoxy-1,2-benzisoxazol-4-yl)phenyl]-N'-(3-chlorophenyl)urea;
   N-[4-(3-amino-7-methoxy-1,2-benzisoxazol-4-yl)phenyl]-N'-(2-fluoro-5-methylphenyl)urea;
   N-{4-[3-amino-7-(4-morpholinylmethyl)-1,2-benzisoxazol-4-yl]phenyl}-N'-[2-fluoro-5-(trifluoromethyl)phenyl]urea;
   N-{4-[3-amino-7-(4-morpholinylmethyl)-1,2-benzisoxazol-4-yl]phenyl}-N'-[3-(trifluoromethyl)phenyl]urea;
   N-{4-[3-amino-7-(4-morpholinylmethyl)-1,2-benzisoxazol-4-yl]phenyl}-N'-(3-chlorophenyl)urea;
   N-{4-[3-amino-7-(4-morpholinylmethyl)-1,2-benzisoxazol-4-yl]phenyl}-N'-(3-methylphenyl)urea;
   N-{4-[3-amino-7-(4-morpholinylmethyl)-1,2-benzisoxazol-4-yl]phenyl}-N'-(2-fluoro-5-methylphenyl)urea;
   N-{4-[3-amino-7-(4-morpholinylmethyl)-1,2-benzisoxazol-4-yl]phenyl}-N'-(3,5-dimethylphenyl)urea;
   N-{4-[3-amino-7-(4-morpholinylmethyl)-1,2-benzisoxazol-4-yl]phenyl}-N'-(3-phenoxyphenyl)urea;
   N-{4-[3-amino-7-(4-morpholmy!methyl)-1,2-benzisoxazol-4-yl]phenyl}-N'-(3-bromophenyl)urea;
   N-(4-{3-amino-7-[2-(4-morpholinyl)ethoxy]-1,2-benzisoxazol-4-yl}phenyl)-N'-[3-(trifluoromethyl)phenyl]urea;
   N-(4-{3-amino-7-[2-(4-morpholinyl)ethoxy]-1,2-benzisoxazol-4-yl}phenyl)-N'-(2-fluoro-5-methylphenyl)urea;
   N-(4-{3-amino-7-[2-(4-morpholinyl)ethoxy]-1,2-benzisoxazol-4-yl}phenyl)-N'-[2-fluoro-5-(trifluoromethyl)phenyl]urea;
   N-(4-{3-amino-7-[2-(4-morpholinyl)ethoxy]-1,2-benzisoxazol-4-yl}phenyl)-N'-(3-methylphenyl)urea;
   N-[4-(3-amino-1,2-benzisoxazol-4-yl)phenyl]-N'-(3,5-dimethylphenyl)urea;
   N-[4-(3-amino-1,2-benzisoxazol-4-yl)phenyl]-N'-phenylurea;
   N-[4-(3-amino-1,2-benzisoxazol-4-yl)phenyl]-N'-(4-methylphenyl)urea;
   N-[4-(3-amino-1,2-benzisoxazol-4-yl)phenyl]-N'-(3-cyanophenyl)urea;
   N-[4-(3-amino-1,2-benzisoxazol-4-yl)phenyl]-N'-[4-fluoro-3-(trifluoromethyl)phenyl]urea;
   N-[4-(3-amino-1,2-benzisoxazol-4-yl)phenyl]-N'-(3-bromophenyl)urea;
   N-[4-(3-amino-1,2-benzisoxazol-4-yl)phenyl]-N'-(3-chlorophenyl)urea;
   N-[4-(3-amino-1,2-benzisoxazol-4-yl)phenyl]-N'-(3-ethylphenyl)urea;
   N-[4-(3-amino-1,2-benzisoxazol-4-yl)phenyl]-N'-[4-(trifluoromethyl)phenyl]urea;
   N-[4-(3-amino-1,2-benzisoxazol-4-yl)phenyl]-N'-(3-fluoro-4-methylphenyl)urea;
   N-[4-(3-amino-1,2-benzisoxazol-4-yl)phenyl]-N'-(3-fluorophenyl)urea;
   N-[4-(3-amino-1,2-benzisoxazol-4-yl)phenyl]-N'-(3,5-difluorophenyl)urea;
   N-[4-(3-amino-1,2-benzisoxazol-4-yl)phenyl]-N'-(3-methoxyphenyl)urea;
   N-[4-(3-amino-1,2-benzisoxazol-4-yl)phenyl]-N'-(4-methoxyphenyl)urea;
   N-[4-(3-amino-1,2-benzisoxazol-4-yl)phenyl]urea;
   N-[4-(3-amino-1,2-benzisoxazol-4-yl)phenyl]-N'-(3-nitrophenyl)urea;
   N-[4-(3-amino-1,2-benzisoxazol-4-yl)phenyl]-N'-(4-fluorophenyl)urea;
   N-[4-(3-amino-1,2-benzisoxazol-4-yl)phenyl]-N'-(2-fluorophenyl)urea;
   N-[4-(3-amino-1,2-benzisoxazol-4-yl)phenyl]-N'-(3-chloro-4-fluorophenyl)urea;
   N-[4-(3-amino-1,2-benzisoxazol-4-yl)phenyl]-N'-(3-chloro-4-methoxyphenyl)urea;
   N-[4-(3-amino-1,2-benzisoxazol-4-yl)phenyl]-N'-[4-(dimethylamino)phenyl]urea;
   N-[4-(3-amino-1,2-benzisoxazol-4-yl)phenyl]-N'-[4-(trifluoromethoxy)phenyl]urea;
   N-[4-(3-amino-1,2-benzisoxazol-4-yl)phenyl]-N'-[2-(trifluoromethoxy)phenyl]urea;
   N-[4-(3-amino-1,2-benzisoxazol-4-yl)phenyl]-N'-[3,5-bis(trifluoromethyl)phenyl]urea;
   N-[4-(3-amino-1,2-benzisoxazol-4-yl)phenyl]-N'-(3-chloro-4-methylphenyl)urea;
   N-[4-(3-amino-7-methoxy-1,2-benzisoxazol-4-yl)phenyl]-N'-[3,5-bis(trifluoromethyl)phenyl]urea;
   N-[4-(3-amino-7-methoxy-1,2-benzisoxazol-4-yl)phenyl]-N'-[4-(trifluoromethoxy)phenyl]urea;
   N-[4-(3-amino-7-methoxy-1,2-benzisoxazol-4-yl)phenyl]-N'-(3-fluorophenyl)urea;
   N-[4-(3-amino-7-methoxy-1,2-benzisoxazol-4-yl)phenyl]-N'-(3-methoxyphenyl)urea;
   N-[4-(3-amino-7-methoxy-1,2-benzisoxazol-4-yl)phenyl]-N'-(3,5-difluorophenyl)urea;
   N-[4-(3-amino-7-methoxy-1,2-benzisoxazol-4-yl)phenyl]-N'-(4-methylphenyl)urea;
   N-[4-(3-amino-7-methoxy-1,2-benzisoxazol-4-yl)phenyl]-N'-(3-bromophenyl)urea;
   N-[4-(3-amino-7-methoxy-1,2-benzisoxazol-4-yl)phenyl]-N'-(3,5-dimethylphenyl)urea;
   N-[4-(3-amino-7-methoxy-1,2-benzisoxazol-4-yl)phenyl]-N'-[4-(dimethylamino)phenyl]urea;
   N-[4-(3-amino-7-methyl-1,2-benzisoxazol-4-yl)phenyl]-N'-(3-methylphenyl)urea;
   N-[4-(3-amino-7-methyl-1,2-benzisoxazol-4-yl)phenyl]-N'-(3-chlorophenyl)urea;
   N-[4-(3-amino-7-methyl-1,2-benzisoxazol-4-yl)phenyl]-N'-(2-fluoro-5-methylphenyl)urea;
   N-[4-(3-amino-7-methyl-1,2-benzisoxazol-4-yl)phenyl]-N'-[2-fluoro-5-(trifluoromethyl)phenyl]urea;
   N-[4-(3-amino-7-methyl-1,2-benzisoxazol-4-yl)phenyl]-N'-[3-(trifluoromethyl)phenyl]urea;
   N-[4-(3-amino-7-methyl-1,2-benzisoxazol-4-yl)phenyl]-N'-(3,5-dimethylphenyl)urea;
   N-[4-(3-amino-7-methyl-1,2-benzisoxazol-4-yl)phenyl]-N'-(3-ethylphenyl)urea;
   N-[4-(3-amino-7-methyl-1,2-benzisoxazol-4-yl)phenyl]-N'-(4-methylphenyl)urea;
   N-[4-(3-amino-7-methyl-1,2-benzisoxazol-4-yl)phenyl]-N'-[4-(trifluoromethoxy)phenyl]urea;
   N-[4-(3-amino-7-methyl-1,2-benzisoxazol-4-yl)phenyl]-N'-(3-fluoro-4-methylphenyl)urea;
   N-[4-(3-amino-7-methyl-1,2-benzisoxazol-4-yl)phenyl]-N'-(3-methoxyphenyl)urea;
   N-[4-(3-amino-7-methyl-1,2-benzisoxazol-4-yl)phenyl]-N'-phenylurea;
   N-[4-(3-amino-7-methyl-1,2-benzisoxazol-4-yl)phenyl] -N'-[3,5-bis(trifluoromethyl)phenyl]urea;
   N-[4-(3-amino-7-methyl-1,2-benzisoxazol-4-yl)phenyl]-N'-(3-bromophenyl)urea;
   N-[4-(3-amino-7-methyl-1,2-benzisoxazol-4-yl)phenyl]-N'-(3-fluorophenyl)urea;
   N-[4-(3-amino-7-methoxy-1,2-benzisoxazol-4-yl)phenyl]-N'-[4-fluoro-3-(trifluoromethyl)phenyl]urea;
   N-[4-(3-amino-7-methoxy-1,2-benzisoxazol-4-yl)phenyl]-N'-(4-fluoro-3-methylphenyl)urea;
   N-[4-(3-amino-7-fluoro-1,2-benzisoxazol-4-yl)phenyl]-N'-[3-(trifluoromethyl)phenyl]urea;
   N-[4-(3-amino-7-fluoro-1,2-benzisoxazol-4-yl)phenyl]-N'-(3-chlorophenyl)urea;
   N-[4-(3-amino-7-fluoro-1,2-benzisoxazol-4-yl)phenyl]-N'-[4-fluoro-3-(trifluoromethyl)phenyl]urea;
   N-[4-(3-amino-7-fluoro-1,2-benzisoxazol-4-yl)phenyl]-N'-(3-methylphenyl)urea;
   N-[4-(3-amino-7-fluoro-1,2-benzisoxazol-4-yl)phenyl]-N'-[2-fluoro-5-(trifluoromethyl)phenyl]urea;
   N-[4-(3-amino-7-fluoro-1,2-benzisoxazol-4-yl)phenyl]-N'-(2-fluoro-5-methylphenyl)urea;
   N-{4-[3-amino-7-(trifluoromethoxy)-1,2-benzisoxazol-4-yl]phenyl}-N'-[2-fluoro-5-(trifluoromethyl)phenyl]urea;
   N-{4-[3-amino-7-(trifluoromethoxy)-1,2-benzisoxazol-4-yl]phenyl}-N'-[3-(trifluoromethyl)phenyl]urea;
   N-{4-[3-amino-7-(trifluoromethoxy)-1,2-benzisoxazol-4-yl]phenyl}-N'-(2-fluoro-5-methylphenyl)urea;
   N-{4-[3-amino-7-(trifluoromethoxy)-1,2-benzisoxazol-4-yl]phenyl}-N'-(3-chlorophenyl)urea;
   N-{4-[3-amino-7-(trifluoromethoxy)-1,2-benzisoxazol-4-yl]phenyl}-N'-(3-bromophenyl)urea;
   N-{4-[3-amino-7-(trifluoromethoxy)-1,2-benzisoxazol-4-yl]phenyl}-N'-[4-fluoro-3-(trifluoromethyl)phenyl]urea; and
   N-{4-[3-amino-7-(trifluoromethoxy)-1,2-benzisoxazol-4-yl]phenyl}-N'-(4-fluoro-3-methylphenyl)urea.
78. The method of claim 77, wherein said compound is N-[4-[3-amino-1H-indazol-4-yl]phenyl]-N'-(2-fluoro-5-methylphenyl)urea.
79. The method of claim 57, wherein said composition is administered via a method selected from the group consisting of topical, subconjunctival, periocular, retrobulbar, subtenon, intracameral, intravitreal, intraocular, subretinal, posterior juxtascleral, and suprachoroidal administration.
80. The method of claim 79, wherein the composition is administered via intravitreal or subtenon injection of a solution or suspension.
81. The method of claim 79, wherein the composition is administered via intravitreal or subtenon placement of a device.
82. The method of claim 79, wherein the composition is administered via topical ocular administration of a solution or suspension.
83. The method of claim 79, wherein the composition is administered via posterior juxtascleral administration of a gel.
84. The method of claim 79, wherein the composition is administered via intravitreal administration of a bioerodible implant.
85. A method for inhibiting diabetic retinopathy, said method comprising administering to a patient in need thereof a composition comprising a therapeutically effective amount of a receptor tyrosine kinase inhibitor that blocks tyrosine autophosphorylation of VEGF receptor 1, VEGF receptor 2, VEGF receptor 3, Tie-2, PDGFR, c-KIT, Flt-3, and CSF-1R.
86. The method of claim 85, wherein the receptor tyrosine kinase inhibitor has an IC50 of from 0.1 nM to 250 nM for each of the receptors listed in claim 85.
87. The method of claim 85, wherein the receptor tyrosine kinase inhibitor blocks tyrosine autophosphorylation of Tie-2, PDGFR, and VEGF receptor 2 with an IC50 of from 0.1 nM to 200 nM for each receptor.
88. The method of claim 85, wherein the receptor tyrosine kinase inhibitor has an IC50 of from 0.1 nM to 100 nM for at least six of the receptor listed in claim 85.
89. The method of claim 88, wherein the receptor tyrosine kinase inhibitor has an IC50 of from 0.1 nM to 10 nM for at least four of the receptors listed in claim 85.
90. The method of claim 85, wherein the receptor tyrosine kinase inhibitor blocks tyrosine autophosphorylation of VEGF receptor 2, VEGF receptor 1, PDGFR, and Tie-2.
91. The method of claim 90, wherein the tyrosine kinase inhibitor has an IC50 of from 0.1 nM to 200 nM for each of the receptors listed in claim 90.
92. The method of claim 85, wherein the receptor tyrosine kinase inhibitor blocks tyrosine autophosphorylation of VEGF receptor 2, VEGF receptor 1, and Tie-2.
93. The method of claim 92, wherein the receptor tyrosine kinase inhibitor has an IC50 of from 0.1 nM to 200 nM for each of the receptors listed in claim 92.
94. The method of claim 85, wherein the receptor tyrosine kinase inhibitor blocks tyrosine autophosphorylation of VEGF receptor 2, VEGF receptor 1, and PDGFR.
95. The method of claim 94, wherein the receptor tyrosine kinase inhibitor has an IC50 of from 0.1 nM to 100 nM for each of the receptors listed in claim 94.
96. The method of claim 85, wherein the receptor tyrosine kinase inhibitor blocks tyrosine autophosphorylation of VEGF receptor 2 and Tie-2.
97. The method of claim 96, wherein the receptor tyrosine kinase inhibitor has an IC50 of from 0.1 nM to 200 nM for each of the receptors listed in claim 96.
98. The method of claim 97, wherein the receptor tyrosine kinase inhibitor has an IC50 of less than 10 nM for at least one of the receptors listed in claim 96.
99. The method of claim 85, wherein the receptor tyrosine kinase inhibitor blocks tyrosine autophosphorylation of VEGF receptor 2 and PDGFR.
100. The method of claim 99, wherein the receptor tyrosine kinase inhibitor has an IC50 of from 0.1 nM to 100 nM for each of the receptors listed in claim 99.
101. The method of claim 100, wherein the receptor tyrosine kinase inhibitor has an IC50 of less than 10 nM for at least one of the receptors listed in claim 99.
102. The method of claim 85, wherein the receptor tyrosine kinase inhibitor blocks IS tyrosine autophosphorylation of VEGF receptor 2, Tie-2, and PDGFR.
103. The method of claim 102, wherein the receptor tyrosine kinase inhibitor has an IC50 of between 0.1 nM and 200 nM for each of the receptors listed in claim 102.
104. The method of claim 103, wherein the receptor tyrosine kinase inhibitor has an IC50 of less than 10 nM for at least one of the receptors listed in claim 102.
105. The method of claim 85, wherein the receptor tyrosine kinase inhibitor is selected from the group consisting of
   N-[4-[3-amino-1H-indazol-4-yl)phenyl]-N'-(2-fluoro-5-methylphenyl)urea
   N-[4-(3-amino-1,2-benzisoxazol-4-yl)phenyl]-N'-(3-methylphenyl)urea;
   N-[4-(3-amino-1,2-benzisoxazol-4-yl)phenyl]-N'-[2-(trifluoromethyl)phenyl]urea;
   N-[4-(3-amino-1,2-benzisoxazol-4-yl)phenyl]-N'-(2-fluoro-5-methylphenyl)urea;
   N-[4-(3-amino-1,2-benzisoxazol-4-yl)phenyl]-N'-[3-(trifluoromethyl)phenyl]urea;
   N-[4-(3-amino-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-(3-methylphenyl)urea;
   N-[4-(3-amino-7-methoxy-1,2-benzisoxazol-4-yl)phenyl]-N'-[3-(trifluoromethyl)phenyl]urea;
   N-[4-(3-amino-7-methoxy-1,2-benzisoxazol-4-yl)phenyl]-N'-(3-chlorophenyl)urea;
   N-[4-(3-amino-7-methoxy-1,2-benzisoxazol-4-yl)phenyl]-N'-(2-fluoro-5-methylphenyl)urea;
   N-{4-[3-amino-7-(4-morpholinylmethyl)-1,2-benzisoxazol-4-yl]phenyl} -N'-[2-fluoro-5-(trifluoromethyl)phenyl]urea;
   N-{4-[3-amino-7-(4-morpholinylmethyl)-1,2-benzisoxazol-4-yl]phenyl}-N'-[3-(trifluoromethyl)phenyl]urea;
   N-{4-[3-amino-7-(4-morpholinylmethyl)-1,2-benzisoxazol-4-yl]phenyl}-N'-(3-chlorophenyl)urea;
   N-{4-[3-amino-7-(4-morpholinylmethyl)-1,2-benzisoxazol-4-yl]phenyl} -N'-(3-methylphenyl)urea;
   N-{4-[3-amino-7-(4-morpholinylmethyl)-1,2-benzisoxazol-4-yl]phenyl}-N'-(2-fluoro-5-methylphenyl)urea;
   N-{4-[3-amino-7-(4-morpholinylmethyl)-1,2-benzisoxazol-4-yl]phenyl}-N'-(3,5-dimethylphenyl)urea;
   N-{4-[3-amino-7-(4-morpholinylmethyl)-1,2-benzisoxazol-4-yl]phenyl}-N'-(3-phenoxyphenyl)urea;
   N-{4-[3-amino-7-(4-morpholinylmethyl)-1,2-benzisoxazol-4-yl]phenyl}-N'-(3-bromophenyl)urea;
   N-(4-{3-amino-7-[2-(4-morpholinyl)ethoxy]-1,2-benzisoxazol-4-yl}phenyl)-N'-[3-(trifluoromethyl)phenyl]urea;
   N-(4-{3-amino-7-[2-(4-morpholinyl)ethoxy]-1,2-benzisoxazol-4-yl}phenyl)-N'-(2-fluoro-5-methylphenyl)urea;
   N-(4-{3-amino-7-[2-(4-morpholmyl)ethoxy]-1,2-benzisoxazol-4-yl}phenyl)-N'-[2-fluoro-5-(trifluoromethyl)phenyl]urea;
   N-(4-{3-amino-7-[2-(4-morpholinyl)ethoxy]-1,2-benzisoxazol-4-yl}phenyl)-N'-(3-methylphenyl)urea;
   N-[4-(3-amino-1,2-benzisoxazol-4-yl)phenyl]-N'-(3,5-dimethylphenyl)urea;
   N-[4-(3-amino-1,2-benzisoxazol-4-yl)phenyl]-N'-phenylurea;
   N-[4-(3-amino-1,2-benzisoxazol-4-yl)phenyl]-N'-(4-methylphenyl)urea;
   N-[4-(3-amino-1,2-benzisoxazol-4-yl)phenyl]-N'-(3-cyanophenyl)urea;
   N-[4-(3-amino-1,2-benzisoxazol-4-yl)phenyl]-N'-[4-fluoro-3-(trifluoromethyl)phenyl]urea;
   N-[4-(3-amino-1,2-benzisoxazol-4-yl)phenyl]-N'-(3-bromophenyl)urea;
   N-[4-(3-amino-1,2-benzisoxazol-4-yl)phenyl]-N'-(3-chlorophenyl)urea;
   N-[4-(3-amino-1,2-benzisoxazol-4-yl)phenyl]-N'-(3-ethylphenyl)urea;
   N-[4-(3-amino-1,2-benzisoxazol-4-yl)phenyl]-N'-[4-(trifluoromethyl)phenyl]urea;
   N-[4-(3-amino-1,2-benzisoxazol-4-yl)phenyl]-N'-(3-fluoro-4-methylphenyl)urea;
   N-[4-(3-amino-1,2-benzisoxazol-4-yl)phenyl]-N'-(3-fluorophenyl)urea;
   N-[4-(3-amino-1,2-benzisoxazol-4-yl)phenyl]-N'-(3,5-difluorophenyl)urea;
   N-[4-(3-amino-1,2-benzisoxazol-4-yl)phenyl]-N'-(3-methoxyphenyl)urea;
   N-[4-(3-amino-1,2-benzisoxazol-4-yl)phenyl]-N'-(4-methoxyphenyl)urea;
   N-[4-(3-amino-1,2-benzisoxazol-4-yl)phenyl]urea;
   N-[4-(3-amino-1,2-benzisoxazol-4-yl)phenyl]-N'-(3-nitrophenyl)urea;
   N-[4-(3-amino-1,2-benzisoxazol-4-yl)phenyl]-N'-(4-fluorophenyl)urea;
   N-[4-(3-amino-1,2-benzisoxazol-4-yl)phenyl]-N'-(2-fluorophenyl)urea;
   N-[4-(3-amino-1,2-benzisoxazol-4-yl)phenyl]-N'-(3-chloro-4-fluorophenyl)urea;
   N-[4-(3-amino-1,2-benzisoxazol-4-yl)phenyl]-N'-(3-chloro-4-methoxyphenyl)urea;
   N-[4-(3-amino-1,2-benzisoxazol-4-yl)phenyl]-N'-[4-(dimethylamino)phenyl]urea;
   N-[4-(3-amino-1,2-benzisoxazol-4-yl)phenyl]-N'-[4-(trifluoromethoxy)phenyl]urea;
   N-[4-(3-amino-1,2-benzisoxazol-4-yl)phenyl]-N'-[2-(trifluoromethoxy)phenyl]urea;
   N-[4-(3-amino-1,2-benzisoxazol-4-yl)phenyl]-N'-[3,5-bis(trifluoromethyl)phenyl]urea;
   N-[4-(3-amino-1,2-benzisoxazol-4-yl)phenyl]-N'-(3-chloro-4-methylphenyl)urea;
   N-[4-(3-amino-7-methoxy-1,2-benzisoxazol-4-yl)phenyl]-N'-[3,5-bis(trifluoromethyl)phenyl]urea;
   N-[4-(3-amino-7-methoxy-1,2-benzisoxazol-4-yl)phenyl]-N'-[4-(trifluoromethoxy)phenyl]urea;
   N-[4-(3-amino-7-methoxy-1,2-benzisoxazol-4-yl)phenyl]-N'-(3-fluorophenyl)urea;
   N-[4-(3-amino-7-methoxy-1,2-benzisoxazol-4-yl)phenyl]-N'-(3-methoxyphenyl)urea;
   N-[4-(3-amino-7-methoxy-1,2-benzisoxazol-4-yl)phenyl]-N'-(3,5-difluorophenyl)urea;
   N-[4-(3-amino-7-methoxy-1,2-benzisoxazol-4-yl)phenyl]-N'-(4-methylphenyl)urea;
   N-[4-(3-amino-7-methoxy-1,2-benzisoxazol-4-yl)phenyl]-N'-(3-bromophenyl)urea;
   N-[4-(3-amino-7-methoxy-1,2-benzisoxazol-4-yl)phenyl]-N'-(3,5-dimethylphenyl)urea;
   N-[4-(3-amino-7-methoxy-1,2-benzisoxazol-4-yl)phenyl]-N'-[4-(dimethylamino)phenyl]urea;
   N-[4-(3-amino-7-methyl-1,2-benzisoxazol-4-yl)phenyl]-N'-(3-methylphenyl)urea;
   N-[4-(3-amino-7-methyl-1,2-benzisoxazol-4-yl)phenyl]-N'-(3-chlorophenyl)urea;
   N-[4-(3-amino-7-methyl-1,2-benzisoxazol-4-yl)phenyl]-N'-(2-fluoro-5-methylphenyl)urea;
   N-[4-(3-amino-7-methyl-1,2-benzisoxazol-4-yl)phenyl]-N'-[2-fluoro-5-(trifluoromethyl)phenyl]urea;
   N-[4-(3-amino-7-methyl-1,2-benzisoxazol-4-yl)phenyl]-N'-[3-(trifluoromethyl)phenyl]urea;
   N-[4-(3-amino-7-methyl-1,2-benzisoxazol-4-yl)phenyl]-N'-(3,5-dimethylphenyl)urea;
   N-[4-(3-amino-7-methyl-1,2-benzisoxazol-4-yl)phenyl]-N'-(3-ethylphenyl)urea;
   N-[4-(3-amino-7-methyl-1,2-benzisoxazol-4-yl)phenyl]-N'-(4-methylphenyl)urea;
   N-[4-(3-amino-7-methyl-1,2-benzisoxazol-4-yl)phenyl]-N'-[4-(trifluoromethoxy)phenyl]urea;
   N-[4-(3-amino-7-methyl-1,2-benzisoxazol-4-yl)phenyl]-N'-(3-fluoro-4-methylphenyl)urea;
   N-[4-(3-amino-7-methyl-1,2-benzisoxazol-4-yl)phenyl]-N'-(3-methoxyphenyl)urea;
   N-[4-(3-amino-7-methyl-1,2-benzisoxazol-4-yl)phenyl]-N'-phenylurea;
   N-[4-(3-amino-7-methyl-1,2-benzisoxazol-4-yl)phenyl]-N'-[3,5-bis(trifluoromethyl)phenyl]urea;
   N-[4-(3-amino-7-methyl-1,2-benzisoxazol-4-yl)phenyl]-N'-(3-bromophenyl)urea;
   N-[4-(3-amino-7-methyl-1,2-benzisoxazol-4-yl)phenyl]-N'-(3-fluorophenyl)urea;
   N-[4-(3-amino-7-methoxy-1,2-benzisoxazol-4-yl)phenyl]-N'-[4-fluoro-3-(trifluoromethyl)phenyl]urea;
   N-[4-(3-amino-7-methoxy-1,2-benzisoxazol-4-yl)phenyl]-N'-(4-fluoro-3-methylphenyl)urea;
   N-[4-(3-amino-7-fluoro-1,2-benzisoxazol-4-yl)phenyl]-N'-[3-(trifluoromethyl)phenyl]urea;
   N-[4-(3-amino-7-fluoro-1,2-benzisoxazol-4-yl)phenyl]-N'-(3-chlorophenyl)urea;
   N-[4-(3-amino-7-fluoro-1,2-benzisoxazol-4-yl)phenyl]-N'-[4-fluoro-3-(trifluoromethyl)phenyl]urea;
   N-[4-(3-amino-7-fluoro-1,2-benzisoxazol-4-yl)phenyl]-N'-(3-methylphenyl)urea;
   N-[4-(3-amino-7-fluoro-1,2-benzisoxazol-4-yl)phenyl]-N'-[2-fluoro-5-(trifluoromethyl)phenyl]urea;
   N-[4-(3-amino-7-fluoro-1,2-benzisoxazol-4-yl)phenyl]-N'-(2-fluoro-5-methylphenyl)urea;
   N-{4-[3-amino-7-(trifluoromethoxy)-1,2-benzisoxazol-4-yl]phenyl}-N'-[2-fluoro-5-(trifluoromethyl)phenyl]urea;
   N-{4-[3-amino-7-(trifluoromethoxy)-1,2-benzisoxazol-4-yl]phenyl}-N'-[3-(trifluoromethyl)phenyl]urea;
   N-{4-[3-amino-7-(trifluoromethoxy)-1,2-benzisoxazol-4-yl]phenyl}-N'-(2-fluoro-5-methylphenyl)urea;
   N-{4-[3-amino-7-(trifluoromethoxy)-1,2-benzisoxazol-4-yl]phenyl}-N'-(3-chlorophenyl)urea;
   N-{4-[3-amino-7-(trifluoromethoxy)-1,2-benzisoxazol-4-yl]phenyl}-N'-(3-bromophenyl)urea;
   N-{4-[3-amino-7-(trifluoromethoxy)-1,2-benzisoxazol-4-yl]phenyl}-N'-[4-fluoro-3-(trifluoromethyl)phenyl]urea; and
   N-{4-[3-amino-7-(trifluoromethoxy)-1,2-benzisoxazol-4-yl]phenyl}-N'-(4-fluoro-3-methylphenyl)urea.
106. The method of claim 105, wherein said compound is N-[4-[3-amino-1H-indazol-4-yl]phenyl]-N'-(2-fluoro-5-methylphenyl)urea.
107. The method of claim 85, wherein said composition is administered via a method selected from the group consisting of topical, subconjunctival, periocular, retrobulbar, subtenon, intracameral, intravitreal, intraocular, subretinal, posterior juxtascleral, and suprachoroidal administration.
108. The method of claim 107, wherein the composition is administered via intravitreal or subtenon injection of a solution or suspension.
109. The method of claim 107, wherein the composition is administered via intravitreal or subtenon placement of a device.
110. The method of claim 107, wherein the composition is administered via topical ocular administration of a solution or suspension.
111. The method of claim 107, wherein the composition is administered via posterior juxtascleral administration of a gel.
112. The method of claim 107, wherein the composition is administered via intravitreal administration of a bioerodible implant.
113. A method for inhibiting sequela associated with retinal ischemia, said method comprising administering to a patient in need thereof a composition comprising a therapeutically effective amount of a receptor tyrosine kinase inhibitor that blocks tyrosine autophosphorylation of VEGF receptor 1, VEGF receptor 2, VEGF receptor 3, Tie-2, PDGFR, c-KIT, Flt-3, and CSF-1R.
114. The method of claim 113, wherein the receptor tyrosine kinase inhibitor has an IC50 of from 0.1 nM to 250 nM for each of the receptors listed in claim 113.
115. The method of claim 113, wherein the receptor tyrosine kinase inhibitor blocks tyrosine autophosphorylation of Tie-2, PDGFR, and VEGF receptor 2 with an IC50 of from 0.1 nM to 200 nM for each receptor.
116. The method of claim 114, wherein the receptor tyrosine kinase inhibitor has an IC50 of from 0.1 nM to 100 nM for at least six of the receptor listed in claim 113.
117. The method of claim 116, wherein the receptor tyrosine kinase inhibitor has an IC50 of from 0.1 nM to 10 nM for at least four of the receptors listed in claim 116.
118. The method of claim 113, wherein the receptor tyrosine kinase inhibitor blocks tyrosine autophosphorylation of VEGF receptor 2, VEGF receptor 1, PDGFR, and Tie-2.
119. The method of claim 118, wherein the tyrosine kinase inhibitor has an IC50 of from 0.1 nM to 200 nM for each of the receptors listed in claim 118.
120. The method of claim 113, wherein the receptor tyrosine kinase inhibitor blocks tyrosine autophosphorylation of VEGF receptor 2, VEGF receptor 1, and Tie-2.
121. The method of claim 120, wherein the receptor tyrosine kinase inhibitor has an IC50 of from 0.1 nM to 200 nM for each of the receptors listed in claim 120.
122. The method of claim 113, wherein the receptor tyrosine kinase inhibitor blocks tyrosine autophosphorylation of VEGF receptor 2, VEGF receptor 1, and PDGFR.
123. The method of claim 122, wherein the receptor tyrosine kinase inhibitor has an IC50 of from 0.1 nM to 100 nM for each of the receptors listed in claim 122.
124. The method of claim 113, wherein the receptor tyrosine kinase inhibitor blocks tyrosine autophosphorylation of VEGF receptor 2 and Tie-2.
125. The method of claim 124, wherein the receptor tyrosine kinase inhibitor has an IC50 of from 0.1 nM to 200 nM for each of the receptors listed in claim 124.
126. The method of claim 125, wherein the receptor tyrosine kinase inhibitor has an IC50 of less than 10 nM for at least one of the receptors listed in claim 124.
127. The method of claim 113, wherein the receptor tyrosine kinase inhibitor blocks tyrosine autophosphorylation of VEGF receptor 2 and PDGFR.
128. The method of claim 127, wherein the receptor tyrosine kinase inhibitor has an IC50 of from 0.1 nM to 100 nM for each of the receptors listed in claim 127.
129. The method of claim 128, wherein the receptor tyrosine kinase inhibitor has an IC50 of less than 10 nM for at least one of the receptors listed in claim 127.
130. The method of claim 113, wherein the receptor tyrosine kinase inhibitor blocks tyrosine autophosphorylation of VEGF receptor 2, Tie-2, and PDGFR.
131. The method of claim 130, wherein the receptor tyrosine kinase inhibitor has an IC50 of between 0.1 nM and 200 nM for each of the receptors listed in claim 130.
132. The method of claim 131, wherein the receptor tyrosine kinase inhibitor has an IC50 of less than 10 nM for at least one of the receptors listed in claim 130.
133. The method of claim 113, wherein the receptor tyrosine kinase inhibitor is selected from the group consisting of
   N-[4-[3-amino-1H-indazol-4-yl)phenyl]-N'-(2-fluoro-5-methylphenyl)urea
   N-[4-(3-amino-1,2-benzisoxazol-4-yl)phenyl]-N'-(3-methylphenyl)urea;
   N-[4-(3-amino-1,2-benzisoxazol-4-yl)phenyl]-N'-[2-(trifluoromethyl)phenyl]urea;
   N-[4-(3-amino-1,2-benzisoxazol-4-yl)phenyl]-N'-(2-fluoro-5-methylphenyl)urea;
   N-[4-(3-amino-1,2-benzisoxazol-4-yl)phenyl]-N'-[3-(trifluoromethyl)phenyl]urea;
   N-[4-(3-amino-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-[2-fluoro-5-(trifluoromethyl)phenyl]urea;
   N-[4-(3-amino-7-methoxy-1,2-benzisoxazol-4-yl)phenyl]-N'-(3-methylphenyl)urea;
   N-[4-(3-amino-7-methoxy-1,2-benzisoxazol-4-yl)phenyl]-N'-[3-(trifluoromethyl)phenyl]urea;
   N-[4-(3-amino-7-methoxy-1,2-benzisoxazol-4-yl)phenyl]-N'-(3-chlorophenyl)urea;
   N-[4-(3-amino-7-methoxy-1,2-benzisoxazol-4-yl)phenyl]-N'-(2-fluoro-5-methylphenyl)urea;
   N-{4-[3-amino-7-(4-morpholinyfmethyl)-1,2-benzisoxazol-4-yl]phenyl}-N'-[2-fluoro-5-(trifluoromethyl)phenyl]urea;
   N-{4-[3-amino-7-(4-morpholinymethyl)-1,2-benzisoxazol-4-yl]phenyl}-N'-[3-(trifluoromethyl)phenyl]urea;
   N-{4-[3-amino-7-(4-morpholinylmethyl)-1,2-benzisoxazol-4-yl]phenyl}-N'-(3-chlorophenyl)urea;
   N-{4-[3-amino-7-(4-morpholinylmethyl)-1,2-benzisoxazol-4-yl]phenyl}-N'-(3-methylphenyl)urea;
   N-{4-[3-amino-7-(4-morpholinylmethyl)-1,2-benzisoxazol-4-yl]phenyl}-N'-(2-fluoro-5-methylphenyl)urea;
   N-{4-[3-amino-7-(4-morpholinylmethyl)-1,2-benzisoxazol-4-yl]phenyl}-N'-(3,5-dimethylphenyl)urea;
   N-{4-[3-amino-7-(4-morpholinylmethyl)-1,2-benzisoxazol-4-yl]phenyl}-N'-(3-phenoxyphenyl)urea;
   N-{4-[3-amino-7-(4-morpholinylmethyl)-1,2-benzisoxazol-4-yl]phenyl}-N'-(3-bromophenyl)urea;
   N-(4-{3-amino-7-[2-(4-morpholinyl)ethoxy]-1,2-benzisoxazol-4-yl}phenyl)-N'-[3-(trifluoromethyl)phenyl]urea;
   N-(4-{3-amino-7-[2-(4-morpholinyl)ethoxy]-1,2-benzisoxazol-4-yl}phenyl)-N'-(2-fluoro-5-methylphenyl)urea;
   N-(4-{3-amino-7-[2-(4-morpholinyl)ethoxy]-1,2-benzisoxazol-4-yl}phenyl)-N'-[2-fluoro-5-(trifluoromethyl)phenyl]urea;
   N-(4-{3-amino-7-[2-(4-morpholinyl)ethoxy]-1,2-benzisoxazol-4-yl}phenyl)-N'-(3-methylphenyl)urea;
   N-[4-(3-amino-1,2-benzisoxazol-4-yl)phenyl]-N'-(3,5-dimethylphenyl)urea;
   N-[4-(3-amino-1,2-benzisoxazol-4-yl)phenyl]-N'-phenylurea;
   N-[4-(3-amino-1,2-benzisoxazol-4-yl)phenyl]-N'-(4-methylphenyl)urea;
   N-[4-(3-amino-1,2-benzisoxazol-4-yl)phenyl]-N'-(3-cyanophenyl)urea;
   N-[4-(3-amino-1,2-benzisoxazol-4-yl)phenyl]-N'-[4-fluoro-3-(trifluoromethyl)phenyl]urea;
   N-[4-(3-amino-1,2-benzisoxazol-4-yl)phenyl]-N'-(3-bromophenyl)urea;
   N-[4-(3-amino-1,2-benzisoxazol-4-yl)phenyl]-N'-(3-chlorophenyl)urea;
   N-[4-(3-amino-1,2-benzisoxazol-4-yl)phenyl]-N'-(3-ethylphenyl)urea;
   N-[4-(3-amino-1,2-benzisoxazol-4-yl)phenyl]-N'-[4-(trifluoromethyl)phenyl]urea;
   N-[4-(3-amino-1,2-benzisoxazol-4-yl)phenyl]-N'-(3-fluoro-4-methylphenyl)urea;
   N-[4-(3-amino-1,2-benzisoxazol-4-yl)phenyl]-N'-(3-fluorophenyl)urea;
   N-[4-(3-amino-1,2-benzisoxazol-4-yl)phenyl]-N'-(3,5-difluorophenyl)urea;
   N-[4-(3-amino-1,2-benzisoxazol-4-yl)phenyl]-N'-(3-methoxyphenyl)urea;
   N-[4-(3-amino-1,2-benzisoxazol-4-yl)phenyl]-N'-(4-methoxyphenyl)urea;
   N-[4-(3-amino-1,2-benzisoxazol-4-yl)phenyl]urea;
   N-[4-(3-amino-1,2-benzisoxazol-4-yl)phenyl]-N'-(3-nitrophenyl)urea;
   N-[4-(3-amino-1,2-benzisoxazol-4-yl)phenyl]-N'-(4-fluorophenyl)urea;
   N-[4-(3-amino-1,2-benzisoxazol-4-yl)phenyl]-N'-(2-fluorophenyl)urea;
   N-[4-(3-amino-1,2-benzisoxazol-4-yl)phenyl]-N'-(3-chloro-4-fluorophenyl)urea;
   N-[4-(3-amino-1,2-benzisoxazol-4-yl)phenyl]-N'-(3-chloro-4-methoxyphenyl)urea;
   N-[4-(3-amino-1,2-benzisoxazol-4-yl)phenyl]-N'-[4-(dimethylamino)phenyl]urea;
   N-[4-(3-amino-1,2-benzisoxazol-4-yl)phenyl]-N'-[4-(trifluoromethoxy)phenyl]urea;
   N-[4-(3-amino-1,2-benzisoxazol-4-yl)phenyl]-N'-[2-(trifluoromethoxy)phenyl]urea;
   N-[4-(3-amino-1,2-benzisoxazol-4-yl)phenyl]-N'-[3,5-bis(trifluoromethyl)phenyl]urea;
   N-[4-(3-amino-1,2-benzisoxazol-4-yl)phenyl]-N'-(3-chloro-4-methylphenyl)urea;
   N-[4-(3-amino-7-methoxy-1,2-benzisoxazol-4-yl)phenyl]-N'-[3,5-bis(trifluoromethyl)phenyl]urea;
   N-[4-(3-amino-7-methoxy-1,2-benzisoxazol-4-yl)phenyl]-N'-[4-(trifluoromethoxy)phenyl]urea;
   N-[4-(3-amino-7-methoxy-1,2-benzisoxazol-4-yl)phenyl]-N'-(3-fluorophenyl)urea;
   N-[4-(3-amino-7-methoxy-1,2-benzisoxazol-4-yl)phenyl]-N'-(3-methoxyphenyl)urea;
   N-[4-(3-amino-7-methoxy-1,2-benzisoxazol-4-yl)phenyl]-N'-(3,5-difluorophenyl)urea;
   N-[4-(3-amino-7-methoxy-1,2-benzisoxazol-4-yl)phenyl]-N'-(4-methylphenyl)urea;
   N-[4-(3-amino-7-methoxy-1,2-benzisoxazol-4-yl)phenyl]-N'-(3-bromophenyl)urea;
   N-[4-(3-amino-7-methoxy-1,2-benzisoxazol-4-yl)phenyl]-N'-(3,5-dimethylphenyl)urea;
   N-[4-(3-amino-7-methoxy-1,2-benzisoxazol-4-yl)phenyl]-N'-[4-(dimethylamino)phenyl]urea;
   N-[4-(3-amino-7-methyl-1,2-benzisoxazol-4-yl)phenyl]-N'-(3-methylphenyl)urea;
   N-[4-(3-amino-7-methyl-1,2-benzisoxazol-4-yl)phenyl]-N'-(3-chlorophenyl)urea;
   N-[4-(3-amino-7-methyl-1,2-benzisoxazol-4-yl)phenyl]-N'-(2-fluoro-5-methylphenyl)urea;
   N-[4-(3-amino-7-methyl-1,2-benzisoxazol-4-yl)phenyl]-N'-[2-fluoro-5-(trifluoromethyl)phenyl]urea;
   N-[4-(3-amino-7-methyl-1,2-benzisoxazol-4-yl)phenyl]-N'-[3-(trifluoromethyl)phenyl]urea;
   N-[4-(3-amino-7-methyl-1,2-benzisoxazol-4-yl)phenyl]-N'-(3,5-dimethylphenyl)urea;
   N-[4-(3-amino-7-methyl-1,2-benzisoxazol-4-yl)phenyl]-N'-(3-ethylphenyl)urea;
   N-[4-(3-amino-7-methyl-1,2-benzisoxazol-4-yl)phenyl]-N'-(4-methylphenyl)urea;
   N-[4-(3-amino-7-methyl-1,2-benzisoxazol-4-yl)phenyl]-N'-[4-(trifluoromethoxy)phenyl]urea;
   N-[4-(3-amino-7-methyl-1,2-benzisoxazol-4-yl)phenyl]-N'-(3-fluoro-4-methylphenyl)urea;
   N-[4-(3-amino-7-methyl-1,2-benzisoxazol-4-yl)phenyl]-N'-(3-methoxyphenyl)urea;
   N-[4-(3-amino-7-methyl-1,2-benzisoxazol-4-yl)phenyl]-N'-phenylurea;
   N- [4-(3 -amino-7-methyl-1,2-benzisoxazol-4-yl)phenyl] -N'-[3,5-bis(trifluoromethyl)phenyl]urea;
   N-[4-(3-amino-7-methyl-1,2-benzisoxazol-4-yl)phenyl]-N'-(3-bromophenyl)urea;
   N-[4-(3-amino-7-methyl-1,2-benzisoxazol-4-yl)phenyl]-N'-(3-fluorophenyl)urea;
   N-[4-(3-amino-7-methoxy-1,2-benzisoxazol-4-yl)phenyl]-N'-[4-fluoro-3-(trifluoromethyl)phenyl]urea;
   N-[4-(3-amino-7-methoxy-1,2-benzisoxazol-4-yl)phenyl]-N'-(4-fluoro-3-methylphenyl)urea;
   N-[4-(3-amino-7-fluoro-1,2-benzisoxazol-4-yl)phenyl]-N'-[3-(trifluoromethyl)phenyl]urea;
   N-[4-(3-amino-7-fluoro-1,2-benzisoxazol-4-yl)phenyl]-N'-(3-chlorophenyl)urea;
   N-[4-(3-amino-7-fluoro-1,2-benzisoxazol-4-yl)phenyl]-N'-[4-fluoro-3-(trifluoromethyl)phenyl]urea;
   N-[4-(3-amino-7-fluoro-1,2-benzisoxazol-4-yl)phenyl]-N'-(3-methylphenyl)urea;
   N-[4-(3-amino-7-fluoro-1,2-benzisoxazol-4-yl)phenyl]-N'-[2-fluoro-5-(trifluoromethyl)phenyl]urea;
   N-[4-(3-amino-7-fluoro-1,2-benzisoxazol-4-yl)phenyl]-N'-(2-fluoro-5-methylphenyl)urea;
   N-{4-[3-amino-7-(trifluoromethoxy)-1,2-benzisoxazol-4-yl]phenyl}-N'-[2-fluoro-5-(trifluoromethyl)phenyl]urea;
   N-{4-[3-amino-7-(trifluoromethoxy)-1,2-benzisoxazol-4-yl]phenyl}-N'-[3-(trifluoromethyl)phenyl]urea;
   N-{4-[3-amino-7-(trifluoromethoxy)-1,2-benzisoxazol-4-yl]phenyl}-N'-(2-fluoro-5-methylphenyl)urea;
   N-{4-[3-amino-7-(trifluoromethoxy)-1,2-benzisoxazol-4-yl]phenyl}-N'-(3-chlorophenyl)urea;
   N-{4-[3-amino-7-(trifluoromethoxy)-1,2-benzisoxazol-4-yl]phenyl}-N'-(3-bromophenyl)urea;
   N-{4-[3-amino-7-(trifluoromethoxy)-1,2-benzisoxazol-4-yl]phenyl}-N'-[4-fluoro-3-(trifluoromethyl)phenyl]urea; and
   N-{4-[3-amino-7-(trifluoromethoxy)-1,2-benzisoxazol-4-yl]phenyl}-N'-(4-fluoro-3-methylphenyl)urea.
134. The method of claim 133, wherein said compound is N-[4-[3-amino-1H-indazol-4-yl]phenyl]-N'-(2-fluoro-5-methylphenyl)urea.
135. The method of claim 113, wherein said composition is administered via a method selected from the group consisting of topical, subconjunctival, periocular, retrobulbar, subtenon, intracameral, intravitreal, intraocular, subretinal, posterior juxtascleral, and suprachoroidal administration.
136. The method of claim 135, wherein the composition is administered via intravitreal or subtenon injection of a solution or suspension.
137. The method of claim 135, wherein the composition is administered via intravitreal or subtenon placement of a device.
138. The method of claim 135, wherein the composition is administered via topical ocular administration of a solution or suspension.
139. The method of claim 135, wherein the composition is administered via posterior juxtascleral administration of a gel.
140. The method of claim 135, wherein the composition is administered via intravitreal administration of a bioerodible implant.

## Claims

1. Use of a therapeutically effective amount of a receptor tyrosine kinase (RTK) inhibitor that blocks tyrosine autophosphorylation of VEGF receptor 2 with an IC₅₀ of ≤ 10 nM, VEGF receptor 1 with an IC₅₀ of ≤ 10 nM, Tie-2 with an IC₅₀ of ≤ 200 nM, and PDGFR with an IC₅₀ of ≤ 100 nM, wherein the composition is administered via a method selected from the group consisting of subconjunctival, periocular, retrobulbar, subtenon, intracameral, intravitreal, intraocular, subretinal, posterior juxtascleral, and suprachoroidal administration, for inhibiting ocular neovascularization.

2. The use of claim 1, wherein said compound is N-[4-[3-amino-1H-indazol-4-yl]phenyl]-N'-(2-fluoro-5-methylphenyl)urea.

3. The use of claim 1, wherein the composition is administered via intravitreal or subtenon injection of a solution or suspension.

4. The use of claim 1, wherein the composition is administered via intravitreal or subtenon placement of a device.

5. (currently amended) The use of claim 1, wherein the composition is administered via intravitreal administration of a bioerodible implant.

6. Use of a therapeutically effective amount of a receptor tyrosine kinase (RTK) inhibitor that blocks tyrosine autophosphorylation VEGF receptor 2 with an IC₅₀ of ≤ 10 nM, VEGF receptor 1 with an IC₅₀ of ≤ 10 nM Tie-2 with an IC₅₀ of ≤ 200 nM, and PDGFR with an IC₅₀ of ≤ 100 nM, wherein the composition is administered via a method selected from the group consisting of subconjunctival, periocular, retrobulbar, subtenon, intracameral, intravitreal, intraocular, subretinal, posterior juxtascleral, and suprachoroidal administration, for causing regression of neovascularization.

7. The use of claim 6, wherein said compound is N-[4-[3-amino-1H-indazol-4-yl]phenyl]-N'-(2-fluoro-5-methylphenyl)urea.

8. The use of claim 6, wherein the composition is administered via intravitreal or subtenon injection of a solution or suspension.

9. The use of claim 6, wherein the composition is administered via intravitreal or subtenon placement of a device.

10. The use of claim 6, wherein the composition is administered via intravitreal administration of a bioerodible implant.

11. Use of a therapeutically effective amount of a receptor tyrosine kinase inhibitor that blocks tyrosine autophosphorylation of VEGF receptor 2 with an IC₅₀ of ≤ 10 nM, VEGF receptor 1 with an IC₅₀ of ≤ 10 nM and PDGFR with an IC₅₀ of ≤ 100 nM, wherein said composition is administered via a method selected from the group consisting of subconjunctival, periocular, retrobulbar, subtenon, intracameral, intravitreal, intraocular, subretinal, posterior juxtascleral, and suprachoroidal administration, for inhibiting retinal edema.

12. The use of claim 11, wherein said compound is N-[4-[3-amino-1H-indazol-4-yl]phenyl]-N'-(2-fluoro-5-methylphenyl)urea.

13. The use of claim 11, wherein the composition is administered via intravitreal or subtenon injection of a solution or suspension.

14. The use of claim 11, wherein the composition is administered via intravitreal or subtenon placement of a device.

15. Use of a therapeutically effective amount of a receptor tyrosine kinase inhibitor that blocks tyrosine autophosphorylation of VEGF receptor 1, VEGF receptor 2, VEGF receptor 3, Tie-2, PDGFR, c-KIT, Flt-3, and CSF-1R, for inhibiting diabetic retinopathy.

16. The use of claim 15, wherein said compound is N-[4-[3-amino-1H-indazol-4-yl]phenyl]-N'-(2-fluoro-5-methylphenyl)urea.

17. The use of claim 15, wherein the composition is administered via intravitreal or subtenon injection of a solution or suspension.

18. The use of claim 15, wherein the composition is administered via intravitreal or subtenon placement of a device.
